(19) **Europäisches Patentamt**
**European Patent Office**
**Office européen des brevets**

(11) **EP 1 967 582 A1**

(12) **EUROPEAN PATENT APPLICATION**
published in accordance with Art. 158(3) EPC

(43) Date of publication:
**10.09.2008 Bulletin 2008/37**

(51) Int Cl.:
*C12N 5/00* (2006.01)     *C12N 5/06* (2006.01)
*C07K 7/04* (2006.01)

(21) Application number: **06835151.9**

(22) Date of filing: **25.12.2006**

(86) International application number:
**PCT/JP2006/325704**

(87) International publication number:
**WO 2007/074747 (05.07.2007 Gazette 2007/27)**

(84) Designated Contracting States:
**CH DE FR GB LI SE**

(30) Priority: **26.12.2005 JP 2005373070**

(71) Applicant: **Kuraray Co., Ltd.**
**Okayama 710-8622 (JP)**

(72) Inventors:
• **FUJITA, Akio**
**Kurashiki-shi, Okayama 710-8622 (JP)**

• **HOITTA, Yuji**
**Kurashiki-shi, Okayama 710-8622 (JP)**
• **OKA, Keiko**
**Tokyo 100-8115 (JP)**
• **TAKAGI, Michiyo**
**Kurashiki-shi, Okayama 710-8622 (JP)**

(74) Representative: **HOFFMANN EITLE**
**Patent- und Rechtsanwälte**
**Arabellastrasse 4**
**81925 München (DE)**

(54) **MATERIAL FOR CELL CULTURE**

(57)     A ligand construct containing at least two, identical or different, ligand molecules which are capable of binding to a cell surface receptor, the ligand molecules being coupled via a spacer, wherein the ligand construct has at least one functional group for binding substrates. According to the ligand construct of the present invention, the immobilization of the ligand construct to various artificial substrates is facilitated, so that an artificial matrix can be easily prepared on an optimal substrate, whereby cells can be stably cultured.

EP 1 967 582 A1

SEQUENCE LISTING

<110> Kuraray Co., Ltd.

<120> A cell-culturing material

<130> 06-043-PCTJP

<150> JP 2005-373070
<151> 2005-12-26

<160> 15

<170> PatentIn version 3.3

<210> 1
<211> 10
<212> PRT
<213> Artificial

<220>
<223> Ligand

<400> 1

Arg Gly Asp Ser Pro Ala Ser Ser Lys Pro
1               5               10

<210> 2
<211> 11
<212> PRT
<213> Artificial

<220>
<223> Ligand

<400> 2

Gly Arg Gly Asp Ser Pro Ala Ser Ser Lys Gly
1               5               10

<210>  3
<211>  11
<212>  PRT
<213>  Artificial

<220>
<223>  Ligand

<400>  3

Gly Cys Gly Tyr Gly Arg Gly Asp Ser Pro Gly
1               5               10

<210>  4
<211>  18
<212>  PRT
<213>  Artificial

<220>
<223>  Ligand

<400>  4

Gly Gly Gly Pro His Ser Arg Asn Gly Gly Gly Gly Gly Gly Arg Gly
1           5               10              15

Asp Gly

<210> 5
<211> 10
<212> PRT
<213> Artificial

<220>
<223> Ligand

<400> 5

Arg Asn Ile Ala Glu Ile Ile Lys Asp Ala
1               5                    10


<210> 6
<211> 13
<212> PRT
<213> Artificial

<220>
<223> Ligand

<400> 6

Arg Gln Val Phe Gln Val Ala Tyr Ile Ile Ile Lys Ala
1               5                    10


<210> 7
<211> 12
<212> PRT
<213> Artificial

<220>
<223> Ligand

<400> 7

Arg Lys Arg Leu Gln Val Gln Leu Ser Ile Arg Thr
1               5                   10


<210> 8
<211> 14
<212> PRT
<213> Artificial

<220>
<223> Ligand

<400> 8

Gly Pro Ala Gly Gly Lys Asp Gly Glu Ala Gly Ala Gln Gly
1               5                   10


<210> 9
<211> 15
<212> PRT
<213> Artificial

<220>
<223> Ligand

<400> 9

Val Ser Trp Phe Ser Arg His Arg Tyr Ser Pro Phe Ala Val Ser
1               5                   10                  15

```
<210>  10
<211>  13
<212>  PRT
<213>  Artificial

<220>
<223>  Ligand

<400>  10

Gly Tyr Arg Asp Gly Tyr Ala Gly Pro Ile Leu Tyr Asn
1               5                   10


<210>  11
<211>  19
<212>  PRT
<213>  Artificial

<220>
<223>  Ligand

<400>  11

Tyr Glu Lys Pro Gly Ser Pro Pro Arg Glu Val Val Pro Arg Pro Arg
1               5                   10                  15

Pro Gly Val


<210>  12
<211>  22
<212>  PRT
```

<213> Artificial

<220>
<223> Ligand
<400> 12

Arg Pro Ser Leu Ala Lys Lys Gln Arg Phe Arg His Arg Asn Arg Lys
1               5                   10                  15

Gly Tyr Arg Ser Gln Arg
                20


<210> 13
<211> 17
<212> PRT
<213> Artificial

<220>
<223> Ligand

<400> 13

Arg Ile Gln Asn Leu Leu Lys Ile Thr Asn Leu Arg Ile Lys Phe Val
1               5                   10                  15

Lys


<210> 14
<211> 20
<212> PRT
<213> Artificial

<220>

<223> Ligand

<400> 14

Asn Ser Val Asn Ser Lys Ile Pro Lys Ala Cys Cys Val Pro Thr Glu

Leu Ser Ala Ile

<210> 15

<211> 12

<212> PRT

<213> Artificial

<220>

<223> Ligand

<400> 15

Asp Ile Thr Trp Asp Gln Leu Trp Asp Leu Met Lys

**Description**

TECHNICAL FIELD

The present invention relates to a cell culture material capable of regulating a cell function such as adhesion, proliferation, differentiation, undifferentiation, survival, and/or cell death. More specifically, the present invention relates to a ligand construct containing a functional group to be immobilized to a substrate, and being capable of arranging ligand molecules capable of binding to a cell receptor in the nanometer order, and a cell culture substrate containing the construct and a substrate moiety, the construct being immobilized to the substrate moiety via the functional group.

BACKGROUND ART

In recent years, trials for evaluating various substances using cells or trials of applying cells to a therapy have been well performed. The trials for evaluating various substances using cells include, for example, the developments of a system for efficiently performing a test on efficacy, pharmacology, or toxicity of a drug, an evaluation system utilizing cells which substitutes an animal experiment, various sensors using cells, a system for searching a method of amplifying cells and a mechanism for differentiation, or the like (see, for example, Non-Patent Publication 1).

In addition, the trials of applying cells to medicine include, for example, the development of; a cell (regenerative) therapy in which cells cultured extracorporeally are transplanted to be treated, and a scaffold (a biodegradable material serving as footing of cells) is intracorporeally embedded together with the cells to regenerate the defective parts; a diagnostic method for providing an optimal medicine to individuals by evaluating extracorporeally a bio-artificial organ or the cells of patient individuals; and the like (see, for example, Non-Patent Publication 2).

In order to utilize cells for applications as described above, a technique of precisely and accurately regulating a function of cells including, for example, adhesion, proliferation, differentiation, undifferentiation, cell death, or the like, extracorporeally, namely on an artificial substrate, is necessitated. Use of cells which have lost their inherent nature could lead to a fatal detect in the above applications including, for example, that the toxicity of a drug is erroneously understood, that the transplanted cells do not function in a living body, or the like. In order to overcome the disadvantages described above, trials in which artificial substrates are variously modified have been actively carried out. In that instance, ligand molecules having cell adhesion, capable of recognizing cells, especially a cell surface receptor, bears an important function.

Studies have been made from earlier days on regulation of a cell function by coating an artificial substrate with collagen, elastin, fibronectin, laminin or the like, that is an extracellular matrix extracted from a living body as a ligand molecule to reproduce an environment equivalent to one in a living body (see, for example, Non-Patent Publications 2 and 3). Similar trials have been also made in a method of coating an artificial substrate using hormones and growth factors as ligand molecules (see, for example, Non-Patent Publication 4).

However, there are some disadvantages in the method of regulating the function of cells by an extracted extracellular matrix that the kinds of the extracellular matrices that can be purified from a living body are limited, that an extracellular matrix of a very small amount of which extraction would be difficult cannot be employed, and that it is difficult to obtain results of even higher reproducibility by the influences of purity, impurity, or the like. Therefore, it has been difficult to design and reconstruct on an artificial substrate an environment matching each of the functions of individual cells.

In order to overcome the disadvantages described above, namely in order to understand the actions of an extracellular matrix by simplification, and further to overcome the disadvantage of admixture of a pathogen accompanying an extract matrix as a transplanting material, trials of regulating cell function have been made by using a synthetic peptide molecule (for example, an amino acid sequence such as RGD, REDV, IKVAV, or YIGSR when expressed by a single character denotation) contained in an extracellular matrix such as collagen, fibronectin, or laminin, the synthetic peptide molecule being capable of being recognized by a cell surface receptor as a ligand molecule, thereby coating on the artificial substrate the synthetic peptide molecule with variously changing densities and kinds (see, for example, Non-Patent Publications 5 and 6). However, it has been difficult to sufficiently regulate the cell function by merely coating the artificial substrate with a simple synthetic peptide molecule.

In the form of combination with the method as mentioned above, trials of regulating cell functions by interaction of the cells on a finely worked substrate surface have been made. For example, trials of regulating cell functions by arranging ligand molecules by using a technique such as inkjet lithography method (see, for example, Patent Publications 1 and 2), photolithography method (see, for example, Patent Publications 3 and 4, and Non-Patent Publication 7), or microcontact printing method (see, for example, Patent Publication 5 and 6, and Non-Patent Publication 8). In these methods by fine working, a main purpose is to regulate the arrangement or the like of the cells themselves. Therefore, the ligand molecules coating an artificial substrate are extracted extracellular matrices or synthetic peptide molecules, so that it cannot be as a matter of course said that the functions of the cells could be sufficiently regulated.

On the other hand, for example, DA. Di Lullo et al. report that ligand sites that can bind to various cell surface receptors are arranged on a type I collagen molecule chain having a length of about 300 nm in the nanometer order (see, for example, Non-Patent Publication 9). According to this technical idea, it is considered to be difficult to derive sufficient cell functions by the conventional methods of regulating cell functions, such as a method of immobilizing the ligand molecules as mentioned above on an artificial substrate by simply varying binding densities and the kinds of the ligand molecules, a method of combining the above method with fine working in the micrometer order, or the like.

In recent years, trials of regulating cell functions by a structure of which nanostructure is regulated have begun to be carried out. A group of ASG. Curtis et al. have succeeded in the regulation of surface nanotopography by phase separation between polymers, and have progressed in the studies on the correlation between the nanostructure and the cell functions (see, for example, Patent Publication 7 and Non-Patent Publication 10). However, this technique merely regulates a dent-and-projection structure of the substrate surface on a nanoscale, so that it would be difficult to arrange various ligands on the nanoscale.

Trials of regulating functions by contacting cells with nanofibers prepared by an electrospinning method or the like have been recently reported (see, for example, Non-Patent Publication 11). However, this method also merely regulates the dent-and-projection of a substrate surface on a nanoscale, so that any given ligand molecules cannot be arranged on the nanoscale.

As a method of locating a given substance at a given position on a nanometer scale, a dip-pen nanolithography, which is a technique of dipping a probe of an atomic force microscope in a solution of a protein or the like and subjecting a substrate to lithography on a nanometer scale has been proposed (see, for example, Non-Patent Publications 12 and 13). However, even with this technique, a line width and a dot diameter are from 30 to 50 nm, so that it is difficult to reproduce the preparation of an arrangement of the ligand molecules on a nanoscale, as observed in the collagen molecules, from the viewpoint fineness in quality. Moreover, it can be said in this dip-pen nanolithography that it is difficult to subject a substrate to lithography in a wide range suitable for cell culture, so that its actual use would have been difficult at present.

In the technique of fine working described above, as a method capable of realizing an arrangement of ligand molecules with limitations on a nanoscale, a technique of coupling ligand molecules with a polymeric spacer having a nanometer length (spacer method) has been reported. W Dai et al have reported that a ligand construct containing RGD molecules, which are ligand molecules of cell surface receptors contained in fibronectin, or YIGSR molecules, which are ligand molecules in laminin, wherein the ligand molecules themselves are bimolecularly bonded via a polyethylene glycol chain having a molecular weight of about 3500, can be utilized as a cell aggregating agent (see, for example, Non-Patent Publication 14).

In addition, Kawasaki et al. have synthesized a ligand construct containing RGD, which is a ligand molecule of cell surface receptors contained in fibronectin, and PHSRN, a synergy sequence thereof, wherein the RGD and PHSRN are bound via a polyethylene glycol chain having an average molecular weight of from about 2500 to about 3400. This molecule has been immobilized on a dish, and the condition of extension of the cells has been observed (see, for example, Non-Patent Publications 15 and 16). A molecule containing RGD and EILDV, which are ligand molecules in fibronectin bound to each other, or a molecule containing PDSGR and YIGSR, which are ligand molecules in laminin bound to each other, is synthesized in the same manner as above, and used in a test for suppressing metastasis of cancer (see, for example, Non-Patent Publications 17 and 18).

Patent Publication 1: Japanese Patent Laid-Open No. 2002-355025
Patent Publication 2: Japanese Patent Laid-Open No. 2002-355026
Patent Publication 3: Japanese Patent Laid-Open No. Hei-7-308186
Patent Publication 4: Japanese Patent Laid-Open No. Hei-11-151086
Patent Publication 5: Japanese Patent Laid-Open No. 2002-355031
Patent Publication 6: Japanese Unexamined Patent Publication No. 2002-509001
Patent Publication 7: Japanese Unexamined Patent Publication No. 2002-505907
Non-Patent Publication 1: Edited by Tomohisa Ishikawa and Toru Horie, Soyaku Saiensu no Susume-Posutogenomujidai no Paradaimu Shifuto (Recommendations on Pharmaceutical Science-Paradigm Shift of Post-Genome Era), KYORITSU SHUPPAN CO., LTD. (2002)
Non-Patent Publication 2: PP. Lanza, R. Langer, J. Vacanti, Principles of Tissue Engineering, 2nd Ed., Academic Press (2000)
Non-Patent Publication 3: Edited by Akira Koide and Toshihiko Hayashi, Saibogai Matorikkusu-Kiso to Rinsho-(Extracellular Matrix-Fundamentals and Clinical Studies-, AICHI Shuppan Co., Ltd. (2000)
Non-Patent Publication 4: Y. Ito, Biomaterials, 20, 2333 (1999)
Non-Patent Publication 5: U. Hersel, C. Dahmen, H. Kessler, Biomaterials, 24, 4385 (2003)
Non-Patent Publication 6: H. Shin, S. Jo, AG. Mikos, Biomaterials, 24, 4353 (2003)
Non-Patent Publication 7: AS. Blawas, WM. Reichert, Biomaterials, 19, 595-609 (1998)

Non-Patent Publication 8: RS. Kane, S. Takayama, E. Ostuni, DE. Ingber, GM. Whitesides, Biomaterials, 20, 2363-2376 (1999)

Non-Patent Publication 9: GA. Di Lullo, Sm. Sweeney, J. Korkko, L. Alakokko, JD. San Antonio, J. Biol. Chem., 277 (6), 4223-4231 (2002)

Non-Patent Publication 10: MJ. Dalby, D. Giannaras, MO. Riehle, N. Gadegaard, S. Affrossman, ASG. Curtis, Biomaterials, 25, 77-83 (2004)

Non-Patent Publication 11: XM. Mo, CY. Xu, M. Kotaki, S. Ramakrishna, Biomaterials, 25, 1883-1890 (2004)

Non-Patent Publication 12: K-B. Lee, S-J. Park, CA. Mirkin, JC. Smith, M. Mrksich, Science, 295, 1702-1705 (2002)

Non-Patent Publication 13: DL. Wilson, R. Martin, S. Hong, M. Cronin-Golomb, CA. Mirkin, DL. Kaplan, Proc. Nat. Acad. Sci., USA., 98(24), 13660-13664 (2001)

Non-Patent Publication 14: W. Dai, J. Belt, WM. Saltzman, Bio/Technology, 12, 797-801 (1994)

Non-Patent Publication 15: S. Yamamoto, Y. Kaneda, N. Okada, S. Nakagawa, K. Kubo, S. Inoue, M. Maeda, Y. Yamashiro, K. Kawasaki, T. Mayumi, Anti-Cancer Drugs, 5, 424-428 (1994)

Non-Patent Publication 16: Y. Suzuki, K. Hojo, I. Okazaki, H. Kamata, M. Sasaki, M. Maeda, M. Nomizu, Y. Yamamoto, S. Nakagawa, T. Mayumi, K. Kawasaki, Chem. Pharm. Bull. 50(9), 1229-1232 (2002)

Non-Patent Publication 17: M. Maeda, Y. Izuno, K. Kawasaki, y. Kaneda, y. Mu, y. Tsutsumi, KW. Lem, T. Mayumi, Biochem. Biophys. Res. Commun., 241, 595-598 (1997)

Non-Patent Publication 18: M. Maeda, K. Kawasaki, Y. Mu, H. Kamada, Y. Tsutsumi, TJ. Smith, T. Mayumi, Biochem. Biophys. Res. Commun., 248, 485-489 (1998)

DISCLOSURE OF THE INVENTION

PROBLEMS TO BE SOLVED BY THE INVENTION

As described in the prior art exemplified above, as techniques of artificially regulating cell functions, a technique of interacting an artificial environment having a nanostructure and cells, and a technique of nanoarranging of a ligand molecule by using a spacer method have been known. However, upon the preparation of a cell culture substrate, a ligand construct is, so far, bound to a substrate by a mere physical adsorption, there has been a disadvantage that the substrate that can be used is limited. Consequently, an optimal substrate for the cells cannot be selected as long as the conventional techniques exemplified are employed, so that there is a disadvantage that the intended cells cannot be stably cultured.

Accordingly, an object of the present invention is to provide a ligand construct capable of immobilizing to various substrates, and a cell culture substrate containing the construct, wherein the construct is immobilized to the cell culture substrate.

MEANS TO SOLVE THE PROBLEMS

Specifically, the present invention relates to:

[1] a ligand construct containing at least two, identical or different, ligand molecules being capable of binding to a cell surface receptor, the ligand molecules being coupled via a spacer, wherein the ligand construct has at least one functional group for binding substrates; and

[2] a cell culture substrate containing the ligand construct as defined in the above [1] and a substrate moiety, wherein the ligand construct is immobilized to the substrate moiety via a functional group for binding substrates.

EFFECTS OF THE INVENTION

By allowing the ligand construct to have a functional group for binding substrates for the purpose of immobilizing the ligand construct to artificial substrates, the present invention is advantageous in the aspect that the immobilization of the ligand construct to various artificial substrates is facilitated, that an artificial matrix can be easily prepared on an optimal substrate, whereby the cells can be stably cultured, or the like. Therefore, if the ligand construct of the present invention is used, an environment matching each of the functions for each cell can be designed on substrates of various materials and prepared with high reproducibility. According to the cell culture substrate of the present invention, excellent effects that the cell functions can be effectively regulated with high reproducibility, and that a specific signal to the cells, equivalent to an extracellular matrix in a living body, can be provided with high reproducibility, are exhibited.

BEST MODE FOR CARRYING OUT THE INVENTION

The amino acid sequence of a peptide as used herein may be expressed by a single-character denotation or a three-letter denotation of the amino acids as prescribed in the conventionally used biochemical nomenclature. The corresponding relationships between the denotations and the amino acids are as follows: A or Ala: alanine residue; D or Asp: aspartic acid residue; E or Glu: glutamic acid residue; F or Phe: phenylalanine residue; G or Gly: glycine residue; H or His: histidine residue; I or Ile: isoleucine residue; K or Lys: lysine residue; L or Leu: leucine residue; M or Met: methionine residue; N or Asn: asparagine residue; P or Pro: proline residue; Q or Gln: glutamine residue; R or Arg: arginine residue; S or Ser: serine residue; T or Thr: threonine residue; V or Val: valine residue; W or Trp: tryptophan residue; Y or Tyr: tyrosine residue; and C or Cys: cysteine residue.

In the regulation of a cell function such as adhesion or proliferation, a ligand molecule recognized by a cell surface receptor bears a key function. In order to exhibit the function, it is desired that at least two molecules of the ligand molecules are preferably accurately arranged in the nanometer order depending upon the cell functions which the molecules regulate.

The ligand construct of the present invention contains at least two, identical or different ligand molecules, capable of binding to a cell surface receptor, the ligand molecules being coupled via a spacer, and the ligand construct further contains one or more functional groups for binding substrates for immobilizing the ligand construct to a substrate.

In the ligand construct of the present invention, the kinds and the number of the ligand molecules, distances between the ligand molecules, and the like are selected depending upon the cell surface receptor relating to a desired cell regulation.

As to the combinations and the like of the cell surface receptors and the ligand molecules, references may be made to, for example, Saibogai Matorikusu-Kiso to Rinsho- (Extracellular Matrix-Fundamentals and Clinical Studies-, AICHI Shuppan Co., Ltd. (2000), edited by Akira Koide et al.; Masao Hayashi, Jikken Igaku Baio Saiensu, Shin Saibosecchakubunshi no Sekai (Experimental Medicine Bioscience, The World of New Cell Adhesion Molecules), YODOSHA CO., LTD. (2001); Kiyotoshi Sekiguchi and Shintaro Suzuki, Tasaibotai no Kouchiku to Saibo Secchaku Sisutemu (Construct of Multicellular Body and Cell Adhesion System), Shireezu: Baiosaiensu Shinseiki (Series: Bioscience New Century) 8, KYORITSU SHUPPAN CO., LTD. (2001), edited by The Chemical Society of Japan; Jikken Igaku Zokan (Experimental Medicine Extravolume), Saibo Secchaku Kenkyu no Saizensen-Sigunaru Dentatsu kara Gan Ten-I heno Kanyo made- (Frontier of Cell Adhesion Studies-Involvement of Signal Transmission to Metastasis of Cancer), YODOSHA CO., LTD. (1996) edited by Hisataka Sanabe and Shoichiro Tsukita, and the like.

Here, the ligand construct of the present invention is one in which ligand molecules are arranged in the nanometer order, so that the structure thereof may be called "nanostructure."

The cell surface receptor is a receptor existing on a cytoplasmic membrane, and the cell surface receptor is not particularly limited as long as the receptor gives influences to the cell function by binding with ligand molecules. The receptor includes, for example, a receptor for an extracellular matrix, a receptor for intercellular adhesion (which may be also referred to as "intercellular adhesion molecule"), a hormone receptor, a cytokine (growth factor) receptor, and the like. The above-mentioned receptor for intercellular adhesion includes, for example, NCAM, cadherin, selectin, and the like. As the above-mentioned cell surface receptor, an optimal one may be selected in accordance with the cell functions to be regulated.

In the present invention, the phrase "ligand molecules which are capable of binding to a cell surface receptor" refers to synthetic or natural molecules having binding activities to a cell surface receptor, the molecules that give influences to a cell function, for example, adhesion, proliferation, differentiation, undifferentiation, survival and/or cell death. The above-mentioned molecule which is capable of binding a cell surface receptor includes, for example, a synthetic peptide "DITWDQLWDLMK (SEQ ID NO: 15)," and the like, which is a ligand molecule for selectin. In the present invention, by using, for example, molecules which are capable of binding to a receptor for intercellular adhesion as ligand molecules, an excellent effect that the cell-cell interactions can be imitated is exhibited. The essence of the present invention is to provide variously changing the kinds of ligand molecules which are capable of binding to a cell surface receptor, and arrangements thereof on the nanometer order, and a method of giving the most suitable external environment for each of the cells to be treated, and the ligand molecules themselves are not particularly limited.

The above-mentioned ligand molecules are, for example, not particularly limited, together with a cell surface receptor, so long as the object of the present invention is not encumbered. The ligand molecules include, for example, extracellular matrices, molecules which are capable of binding to a receptor for intercellular adhesion, cytokines (growth factors), hormones, antibodies, saccharides, and the like.

The extracellular matrix includes, for example, those described in Saibogai Matorikusu-Kiso to Rinsho- (Extracellular Matrix-Fundamentals and Clinical Studies-, AICHI Shuppan Co., Ltd. (2000), edited by Akira Koide and Toshihiko Hayashi, and the like. The above-mentioned extracellular matrix includes, for example, collagen (also including procollagen), elastin, fibroin, fibronectin, vitronectin, laminin, entactin, tenascin, agrin, Anchorin, thrombospondin, osteopontin, osteocalcin, fibrinogen, proteoglycans, and the like. The proteoglycan includes, for example, aggrecan, agrin, perlecan, dulin, fibromodulin, brevican, and the like. A partial structure derived from the extracellular matrix as described above, in other words, a fragment of a cellular matrix in a living body or a synthetic peptide, may be used as the above-mentioned ligand molecule. In addition, in the present invention, as the above-mentioned ligand molecule, a substance other than those derived from the extracellular matrix may be used. The substance includes an artificially designed and synthesized substance, and the like as substances which are capable of simply binding to a cell surface receptor. Any one of the above-mentioned extracellular matrices may be chemically derivatized as described hereinbelow.

The phrase "a partial structure derived from the extracellular matrix" refers to a fragment obtained by chemically or enzymatically cleaving a part of the extracellular matrix, and purifying the fragment, the purified fragment which is capable of binding to a particular cell surface receptor. The above-mentioned fragment includes, for example, a purified fragment of type I collagen obtained by treatment with CNBr (for example, fragments as described in WD. Staatz, JJ. Walsh, T. Pexton, SA. Santoro, J. Biol. Chem., 265(9), 4778-4781(1990)); a purified fragment of type IV collagen obtained by treatment with CNBr and further, as occasion demands, with a protease such as trypsin, thermolysin, or collagenase (including, for example, fragments having a triple helical structure obtained by the method described in JA. Eble, R. Golbik, K. Mann, K. Kuhn, EMBO J., 12(12), 4795-4802(1993), and further an NC1 domain or the like, obtained by the method as described in EC. Tsilibary, AS. Charonis, J. Cell. Biol., 103(6), 2467-2473(1986)); a cell adhesion fragment obtained by treating fibronectin with a protease such as trypsin or pepsin (see, for example, SK. Akiyama, E. Hasegawa, T. Hasegawa, KM. Yamada, J. Biol. Chem., 260(24), 13256-13260(1985)); and the like. The same can be said for other extracellular matrices, and it is as a matter of course not limited only to the fragments exemplified above.

The phrase "synthetic peptide, which is a partial structure derived from the extracellular matrix" refers to a chemically synthesized oligopeptide in general that is contained in the extracellular matrix, the oligopeptide containing a minimum amino acid sequence which is capable of binding to a particular cell surface receptor. Especially, the oligopeptide is contained in common among fibronectin, vitronectin, laminin, collagen, thrombospondin or the like, as a minimum amino acid sequence which is capable of binding to a cell surface receptor. As the minimum amino acid sequence, the Arg-Gly-Asp (RGD) sequence in which three amino acids are bound has been well known. The synthetic peptide in general containing this RGD can be used as a ligand molecule contained in a ligand construct having a nanostructure in the present invention, and is not particularly limited thereto.

Even more concrete peptide sequences which are linear synthetic peptide containing the RGD sequence as mentioned above that can be used as the ligand molecules in the present invention are not particularly limited, and include, for example, RGD, RGDS, RGDV, RGDT, RGDF, GRGD, GRGDG, GRGDS, GRGDF, GRGDY, GRGDVY, GRGDYPC, GRGDSP, GRGDSG, GRGDNP, GRGDSY, GRGDSPK, YRGDS, YRGDG, YGRGD, CGRGDSY, CGRGDSPK, YAVTGRGDS, RGDSPASSKP (SEQ ID NO: 1), GRGDSPASSKG (SEQ ID NO: 2), GCGYGRGDSPG (SEQ ID NO: 3), GGGPHSRNGGGGGGGRGDG (SEQ ID NO: 4), and the like.

In addition, by cyclizing a peptide containing the RGD sequence, the binding activity to a cell surface receptor can be regulated. The cyclic peptide is not particularly limited, and includes, for example, GPen*GRGDSPC*A, GAC*RGDC*LGA, AC*RGDGWC*G, cyclo(RGDf(NMe)V), cyclo(RGDfV), cyclo(RGDfK), cyclo(RGDEv), cyclo(GRGDfL), cyclo(ARGDfV), cyclo(GRGDfV), and the like. Here, the above-mentioned "Pen" stands for penicillamine, and "* (asterisk)" stands for cysteine residue having disulfide bond in the molecule. Also, the lowercase character contained in a single-character denotation of an amino acid showing a peptide sequence indicates a D-amino acid.

The peptide described above can utilize a minimum amino acid sequence derived from an extracellular matrix of a functional motif contained in common in many of the extracellular matrices in a living body, and the peptide can be contained in a synthetic peptide to be used as a ligand molecule. As the above-mentioned ligand molecule, an optimal synthetic peptide is selected as occasion demands. The ligand molecule includes a minimum amino acid sequence derived from fibronectin including, for example, a synthetic peptide containing a sequence such as NGR, LDV, REDV, EILDV, or KQAGDV, and the like, but not particularly limited thereto.

In addition, the ligand molecule includes a synthetic peptide containing an amino acid sequence derived from laminin, which is one of the extracellular matrices, for example, a sequence of LRGDN, IKVAV, YIGSR, CDPGYIGSR, PDSGR, YFQRYLI, RNIAEIIKDA (SEQ ID NO: 5), or the like. In addition, the above-mentioned ligand molecule is not particularly limited. Regarding laminin, the ligand molecule includes a synthetic peptide, for example, containing a peptide sequence having cell adherent activity as described in Motoyoshi Nomizu, Tanpakushitsu Kakusan Kouso (Proteins, Nucleic Acids and Enzymes), 45(15), 2475-2482(2000), or the like, which is, for example, RQVFQVAYIIIKA (SEQ ID NO: 6) derived from α1 chain, RKRLQVQLSIRT (SEQ ID NO: 7) derived from G domain of α1 chain, or the like.

Similarly, the above-mentioned ligand molecule is not particularly limited, and in the amino acid sequence derived from type I collagen, one of the extracellular matrices, the ligand molecule includes, for example, a synthetic peptide containing, for example, DGEA, KDGEA, GPAGGKDGEAGAQG (SEQ ID NO: 8), GER, GFOGER or the like. In addition, in the present invention, as described in CD. Reyes, AJ. Garcia, J. Biomed. Mater. Res., 65A, 511-523 (2003), a structure in which several peptides containing these amino acid sequences are associated may be used as a ligand molecule. Further, the above-mentioned ligand molecule is not particularly limited, and the peptide sequence derived from elastin includes, for example, VAPG, VGVAPG, VAVAPG, and the like, and the peptide sequence derived from thrombospondin includes, for example, a synthetic peptide containing VTXG. In the present invention, the above-mentioned ligand molecule is not limited to those exemplified above.

An artificially designed and synthesized substance as a substance which is capable of simply binding to a cell surface receptor can be used in a ligand molecule in the present invention, even if the substance is not derived from the extracellular matrix. Here, the ligand molecule, in the event, has two embodiments: An embodiment where a partial structure motif derived from the extracellular matrix is contained, and an embodiment where the partial structure motif is not contained. The ligand molecule includes, for example, a ligand molecule for α5β1 integrin such as C*RRETAWAC* (* is a cysteine residue having a disulfide bond); a ligand molecule for α6β1 integrin such as VSWFSRHRYSPFAVS (SEQ ID NO: 9); and a ligand molecule for αMβ2 integrin such as GYRDGYAGPILYN (SEQ ID NO: 10). Further, an artificially synthesized compound which is capable of binding to a cell surface receptor that is not a peptide described in GA. Sulyok, C. Gibson, SL. Goodman, G. Holzemann, M. Wiesner, H. Kessler, J. Med. Chem., 44(12), 1938-1950 (2001) or the like can be also utilized as the ligand molecule of the present invention.

In addition, among the cell surface receptors, for example, syndecan, NG2, CD44 or the like contains a proteoglycan; therefore, in the present invention, a peptide sequence which is capable of binding to the proteoglycan can be also used as the ligand molecule.

The amino acid sequence which is capable of binding to the proteoglycan includes, for example, consensus sequences such as BBXB, XBBXBX, and further XBBBXXBX (X is a hydrophobic amino acid, and B is a basic amino acid). The amino acid sequence is not particularly limited, and concrete amino acid sequences include, for example, KRSR, FHRRIKA (derived from a bone sialoprotein), PRRARV (derived from fibronectin), WQPPRARI, and the like, and even longer peptide sequences such as YEKPGSPPREVVPRPRPGV (SEQ ID NO: 11) (derived from fibronectin), RPSLAKKQRFRHRNRKGYRSQR (SEQ ID NO: 12) (derived from vitronectin), and RIQNLLKITNLRIKFVK (SEQ ID NO: 13) (derived from laminin).

Besides those exemplified above, linear or cyclic synthetic peptides as described in, for example, U. Hersel, C. Dahmen, H. Kessler, Biomaterials, 24, 4385-4415 (2003); JA. Hubbell, Bio/Technology, 13, 565-576 (1995); H. Shin, S. Jo, AG. Mikos, Biomaterials, 24, 4353-4364 (2003); and E. Koivunen, W. Arap, D. Rajotte, J. Lahdenranta, R. Pasqualini, J. Nuclear Med., 40(5), 883-888(1999) or the like, or references cited therein can be utilized as the ligand molecules in the present invention. In addition, all of the peptide sequences which can be utilized as the ligand molecules in the present invention may be bound with an additional peptide sequence in accordance with its purpose on an N-terminal and/or a C-terminal thereof. The synthetic peptide may contain a non-naturally occurring amino acid, a chemical modified group, one or both of L-form and D-form amino acids, or the like.

The hormone is not particularly limited, and includes, for example, insulin, adrenalin, and the like. The cytokine is not particularly limited, and includes, for example, interleukin, interferon, tumor necrosis factor (TNF), lymphotoxin, colony-stimulating factor (CSF), bone morphogenetic protein (BMP), epidermal growth factor (EGF), nerve growth factor (NGF), insulin-like growth factor (IGF), basic fibroblast growth factor (bFGF), platelet-derived growth factor (PDGF), transforming growth factor (TGF), hematocyte growth factor (HGF), vascular endothelial growth factor (VEGF), and the like. The hormones and cytokines mentioned above can also be utilized as ligand molecules in the present invention, and it is as a matter of course not limited to those exemplified.

Regarding the hormone and the cytokine, a partial structure for which an activity can be expected, contained in those molecules, or an artificially synthesized agonist may be used. As an example, in, for example, a bone morphogenic protein (BMP), if NSVNSKIPKACCVPTELSAI (SEQ ID NO: 14) is used, the same nature as in BMP-2 can be owned (see, for example, Y. Suzuki, M. Tanihara, K. Suzuki, A. Saitou, W. Sufan, Y. Nishimura, J. Biomed. Mater. Res., 50, 405-409(2000)). In addition, similarly, in erythropoietin, the agonist includes YXCXXGPXTWXCXP (X is a replaceable position by several kinds of amino acids.) (see NC. Wrington, FX. Farrell, R. Chang, AK. Kashyap, FP. Barbone, LS. Mulcahy, DL. Johnson, RW. Barrett, LK. Jolliffe, WJ. Dower, Science, 273, 458-463(1996)). However, the example is not limited thereto, and any one of them can be used as the ligand molecule in the present invention.

As the antibody, an antibody against a cell surface receptor, especially preferably a monoclonal antibody, can be used as a ligand molecule in the ligand construct having a nanostructure of the present invention. Antibodies which are capable of binding to various receptors can be utilized, and the antibodies are not limited, and include, for example, various kinds of anti-integrin antibodies, antibodies against cell surface proteoglycans such as syndecan, NG2, and CD44/CSPG, anti-cadherin antibodies, anti-selectin antibodies, and the like. In a case where an antibody is used as a ligand molecule, the antibody itself may be used, or a fragment thereof obtained by a treatment with a protease such as pepsin or papain may be utilized.

Among the intercellular adhesion molecules, NCAM, cadherin or the like is subjected to homophilic binding extracellularly in many cases. Each of the components outside the cytoplasmic membrane is a molecule bound to a receptor for intercellular adhesion. Therefore, the above-mentioned intercellular adhesion molecule bound to the receptor can be used as the above-mentioned ligand molecule. In addition, as the above-mentioned ligand molecules, among the ligand molecules for selectin, a synthetic peptide is exemplified by DITWDQLWDLMK (SEQ ID NO: 15), or the like. As a matter of course, the synthetic peptide is not limited to those exemplified above. The significance of the use of these ligand molecules for cell surface receptors are in the effectiveness upon imitating cell-cell interactions by the ligand construct of the present invention.

As the above-mentioned ligand molecules, saccharides can be also utilized. The above-mentioned saccharides include, for example, saccharides such as glycosaminoglycans contained in proteoglycans, such as hyaluronic acid, chondroitin sulfate, dermatan sulfate, heparan sulfate, heparin, and keratan sulfate. Further, in the present invention, a saccharide which is capable of binding to an asialoglycoprotein receptor on hematocytes, including, for example, galactose, lactose, or a polymerizable lactose monomer which is capable of forming an adhering substrate by polymerization can be utilized as the ligand molecule. In addition, for example, as for selectin, Sialyl Lewis X , an oligosaccharide containing sialic acid and fucose (see, for example, A. Varki, Proc. Natl. Acad. Sci. USA., 91, 7390-7397 (1994)) can be used as the ligand.

As mentioned above, the above-mentioned ligand molecule can be used as the "ligand molecule" in the present invention, so long as the molecule is a substance artificially designed and synthesized to be simply bound to a cell surface receptor, even if the ligand molecule is a substance other than those derived from naturally occurring ligand molecules.

As the ligand molecules contained in the ligand construct having a nanostructure of the present invention, two or more ligand molecules that are optimum for every cell are selected from, for example, extracellular matrices, intercellular adhesion molecules which are capable of binding to a receptor, proliferation factors (growth factors), hormones, antibodies, saccharides, and the like. Here, as the ligand molecule, it is preferable to utilize a fragment of a naturally occurring ligand molecule, a synthetic peptide molecule, a saccharide, an artificially designed and synthesized substance, and the like and complexes thereof, rather than utilizing the naturally occurring ligand molecule itself, from the viewpoint of one of the object of the present invention, in other words, to regulate the arrangement of the ligand molecules in the nanometer order, and provide an optimal environment for each of the cells with excellent reproducibility. Here, although the chain length of a peptide or a sugar is not particularly limited, it is preferable that the chain length is usually from about 3 to about 150 amino acid residues, in a case where the ligand molecule is composed of peptides, and on the other hand, it is preferable that the chain length is about 1 to about 100 monosaccharide residues, in a case where the ligand molecule is composed of sugar chains, from the viewpoint of utilization and effectiveness to regulation of cell functions.

In the ligand construct of the present invention, the above-mentioned ligand molecules are coupled to a spacer described later by a covalent bond or a non-covalent bond. It is preferable that the above-mentioned coupling is carried out by introducing functional groups into each of a ligand molecule and a spacer, and utilizing the functional groups. A functional group to be introduced into the ligand molecule is hereinafter referred to as a "functional group for binding to a spacer," and a functional group to be introduced into the spacer is referred to as a "functional group for binding ligand molecules." The reaction for coupling a spacer and ligand molecules can be selected from various ones as explained hereinbelow. In addition, the functional group for binding to a spacer is not particularly limited. Further, the functional group for binding ligand molecules is also not particularly limited, and an appropriate one may be selected depending upon the functional group for binding to a spacer used.

One of the elements of the present invention is in that a ligand construct having a structure in which two or more ligand molecules as explained above are coupled via a spacer is prepared, and the ligand construct is contacted with the cells, thereby regulating the cell functions. The spacer refers to one that is positioned in an intermediate part between the neighboring ligand molecules, and is capable binding with the neighboring ligand molecules as a single molecule with a given spacing therebetween.

The ligand construct of the present invention comprises at least two, identical or different ligand molecules and a spacer, the ligand molecules and the spacer being coupled, and has an appropriate arrangement of ligand molecules for regulation of cell functions. The spacing (coupling position) between the ligand molecules in one ligand construct, the number of ligand molecules, and the like may be determined depending upon the cells to be regulated, and the cell functions.

The spacing between the ligand molecules is defined by a spacer length, in other words, a length determined in a state where the spacer molecule is stretched. As the spacer length, in other words, the distance between the neighboring ligand molecules, an optimal distance may be selected depending upon each of ligand molecules and each of cells, and the spacer length is preferably within the range of from 1 to 300 nm, and more preferably within the range of from 5 to 100 nm.

As the "spacer" in the present invention, any molecules can be utilized without particular limitation, as long as the spacer is a polymeric compound having a compatibility to cells. Concrete examples thereof include, for example, polyamino acids such as polyglycine, polyalanine, and polysarcosine; polysaccharides such as pullulan, dextran, alginic acid, chitosan, water-soluble cellulose derivatives such as methyl cellulose, carboxymethyl cellulose, and hydroxypropyl cellulose; synthetic polymers such as polyethylene glycol, polypropylene glycol, polyvinyl alcohol, poly(hydroxyethyl methacrylate), polyvinyl pyrrolidone, and polyacrylamide; nucleic acids such as ribonucleic acid, deoxyribonucleic acid, peptide nucleic acid, and derivatives thereof; and combinations thereof.

In order to effectively regulate the cell functions using the ligand construct of the present invention with high reproducibility, it is preferable that the distance between the ligand molecules is defined in an arbitrary distance in the nanometer order. The above-mentioned spacers include as more preferred spacers polyamino acids, polyethylene glycol and polypropylene glycol, and nucleic acids, from the viewpoint of facilitating the specification of the binding site of the ligand molecules, and being capable of even more accurately regulating the distance between the ligand molecules.

In the case of a polyamino acid or a nucleic acid, since monomer units of amino acids or nucleic acids can be stepwise and selectively condensed at the stage of synthesis, accurate specification of the binding site of the ligand molecule is facilitated as explained hereinbelow. On the other hand, among the synthetic polymers, a polyethylene glycol (which may be hereinafter simply referred to as PEG) and a polypropylene glycol (which may be hereinafter simply referred to as PPG) have a functional group which is capable of binding ligand molecules, and the like only at both of the terminals of the polymer chain; therefore, they are excellent in the aspect that the binding site of the ligand molecules can be accurately specified, contrary to an ordinary addition polymerization polymer having a functional group in the side chain. The spacer mentioned above may be branched.

Further, the nucleic acids are even more preferred spacers, from the viewpoint that spacers having substantially the same chain lengths can be prepared, that a complicated higher order structure such as a branched structure can be prepared, that biochemical extension reaction can be applied so that a spacer having a length exceeding 100 nm, while having even lengths can be synthesized, and the like. More concretely, as the spacer composed of nucleic acids (which may be hereinafter simply referred to as a nucleic acid spacer), deoxyribonucleic acid, ribonucleic acid, peptide nucleic acid, or a derivative thereof can be used. The nucleic acid spacer may be composed of a mixture of these.

According to the ligand construct containing a nucleic acid spacer, excellent effects that the distance between the ligand molecules can be substantially evenly set, and also that various artificial matrices which could not have been obtained can be easily prepared, as compared to conventional techniques, by changing the kinds and arrangements of the ligand molecules can be exhibited. By the effects, excellent effects that cell functions can be effectively artificially regulated with high reproducibility, and that an artificial environment having a nanostructure and cells can be interacted are exhibited, whereby specific signals equivalent to those of an extracellular matrix in the living body can be given to cells with high reproducibility.

Deoxyribonucleic acid (which may be hereinafter simply referred to as DNA) and ribonucleic acid (which may be hereinafter simply referred to as RNA) are $3' \rightarrow 5'$ phosphodiester bonded polymers having deoxyribonucleotide and ribonucleotide as a monomer unit. The "peptide nucleic acid (hereinafter also referred to as PNA)" refers to a non-naturally occurring compound having a structure resembling DNA or RNA, as described in, for example, PE. Nielsen, M. Egholm, RH. Berg, O. Buchardt, Science, 254, 1497-1500 (1991). The above-mentioned PNA, more concretely, refers to one forming a backbone with a peptide bond containing 2-aminoethyl-glycine as a monomer unit, not a structure in which nucleotides are bonded with phosphoric diester. By using a polymer having a basal backbone as described above as a spacer in the present invention, a ligand construct having a nanostructure suitable for regulation of cell functions can be provided, where the distance between the ligand molecules is defined to have an arbitrary distance, and the molecular weight of the spacer part is unique. In the nucleic acid spacer, as compared to those spacers of the prior art, since monomer units of nucleotides or the like can be stepwise and selectively condensed, some advantages are in that spacers having substantially the same chain lengths can be prepared that complicated higher order structures such as branched structures can be prepared, that biochemical extension reaction can be applied so that a spacer having a length exceeding 100 nm, even while having even lengths can be synthesized, and the like. The nucleic acid spacer may be composed of a mixture of DNA, RNA, and PNA.

The derivatives of the deoxyribonucleic acid, the ribonucleic acid, the peptide nucleic acid, used as a nucleic acid spacer in the present invention include the ones given hereinbelow.

In the contacting conditions with the cells, in order that the stability of the ligand construct itself of the present invention is increased, that the ligand construct of the present invention is quantitatively analyzed by enzyme immunoassay, fluorescent labeling, or the like, that the ligand construct is labeled with fine particles, to be observed with an electron microscope or the like, and the like, the nucleic acid spacer mentioned above may be subjected to chemical modification. The term "chemical modification" refers to that the main chain of the nucleic acid, such as a phosphate site, a ribose site, or a 2-aminoethyl-glycine site (in the case of PNA) is chemically modified, or a nucleic acid base site or the like is subjected to chemical modification. Examples of chemically modifying the main chain of the nucleic acid include a chemically modified product of phosphorothioate in which resistance to a nuclease is improved by thiolating or the like a phosphate moiety of a structurally unstable deoxyribonucleic acid or ribonucleic acid (although this derivative is not contained in PNA, it is known that the degradation resistance of the PNA itself is inherently high); a chemically modified product into which an antigen site of a chromophore, such as a fluorescein derivative, a rhodamine derivative or Alexa (besides the above, chromophores described in, for example, "non-RI Jikken no Saishin Purotokohru-Keikou no Genri to Jissai: Idenshikaiseki kara Baioime-jingu made" (Latest Protocol on non-RI Experiments - Principle and Practice of Fluorescence: from Genetic Analysis to Bioimaging)," Bessatsu Jikken Igaku (Supplement Experimental Medicine), YODOSHA CO., LTD. (1999) edited by Yasuyuki Kurihara, Hisanari Takeuchi, and Youichi Matsuda, or the like can be exemplified), biotin, bromodeoxyuridine, digoxigenin or the like is introduced (the method of introduction or the like can be exemplified by, for example, one described in Toyozo Takahashi, "DNA Puroubu-Gijutsu to Ohyou- (DNA Probe-Techniques and Applications)" CMC (1988), Toyozo Takahashi, "DNA Puroubu II-Shingijutsu to Shintenkai- (DNA Probe II-New Techniques and New Developments)" CMC (1990), or the like). In addition, as described hereinbelow, the above chemical modification may be provided at the stage where a single-stranded nucleic acid, which serves as a raw material for the nucleic acid spacer, is synthesized. The above-mentioned chemical modifications may be each prepared by the optimal production method, and can be selected depending upon the purposes mentioned above, so that the chemical modifications are not limited to those exemplified above.

The nucleic acid spacer contains a DNA, an RNA, a PNA, or a derivative thereof as mentioned above. Upon the formation of the ligand construct, the structure thereof may be in the state of a single strand, or at least a part of the ligand construct may be in the state of a double helix or triple helix, or a combination thereof, or further one which is branched into 3 or more strands, as described in K. Matsuura, T. Yamashita, Y. Igami, N. Kimizuka, Chem. Commun., 376 (2003); NC. Seeman, Trends in Biotechnology, 17, 437-443 (1999); CM. Niemeyer, Current Opinion in Chemical Biology, 4, 609-618 (2000), JH. Gu, LT. Cai, S. Tanaka, Y. Otsuka, H. Tabata, T. Kawai, J. Appl. Phys., 92, 2816 (2002), or the like. Further, these structural units may be combined to form an even larger primary, secondary, or tertiary structure.

As exemplified above, a single-stranded nucleic acid is preferably used as a raw material for the spacer regardless of what sort of a complicated spacer structure the nucleic acid spacer in the ligand construct takes. A single-stranded nucleic acid can be chemically synthesized to a chain length of up to 150 bases, more practically up to 100 bases, or so (most of the nucleic acids adopts solid phase synthesis method according to phosphamadite method). For example, if a functional group for binding ligand molecules is introduced, for site-specifically introducing the ligand molecules at this stage of chemical synthesis, then the ligand molecules are coupled by binding a functional group for binding to a spacer to the functional group, and whereby the ligand molecules can be properly arranged in the nanometer order with easily keeping a given distance therebetween. The functional group for binding ligand molecules and the functional group for binding to a spacer of the ligand molecules may be bound to each other by forming a covalent bond or a non-covalent bond.

As to the reaction of introducing ligand molecules into a nucleic acid spacer (in other words, a combination of a functional group for binding ligand molecules contained in a nucleic acid spacer and a functional group for binding to a spacer contained in the ligand molecules), in the reaction, a covalent bond or a non-covalent bond may be formed, so long as the nucleic acid spacer and the ligand molecules are stably maintained, so that the reaction of a specific introduction of the intended sites themselves may be carried out.

The reaction of introduction by a covalent bond includes amide bond formation reaction, Michael addition reaction, thioether formation reaction, Diels-Alder cyclization addition reaction, Schiff base formation reaction, reaction of formation of a thiazolidine ring, and the like.

The above-mentioned amide bond formation reaction includes, for example, a method including the step of condensing an amino group and a carboxyl group using a condensing agent such as carbodiimide; a method including the step of reacting an amino group with a carboxyl group-active ester previously activated with N-hydroxysuccinimide or the like; a method including the step of reacting a thioester group and cysteine in which an amino group and a thiol group are remain unreacted to form an amide bond; and the like.

The above-mentioned Michael addition reaction includes, for example, a method including the step of reacting an $\alpha,\beta$-unsaturated carbonyl group such as a maleimide group, an acrylic ester group, an acrylamide group, or a vinylsulfone group, and a thiol group; and the like.

The above-mentioned thioether formation reaction includes, for example, a reaction of a halogenated alkyl group such as a haloacetyl group, an epoxy group, an aziridine group, or the like, and thiol; and the like. The above-mentioned Diels-Alder cyclization addition reaction includes, for example, a method including the step of reacting a conjugated diene such as cyclopentadiene, and an olefin such as quinone; and the like.

The above-mentioned Schiff base formation reaction includes, for example, a method including the step of reacting an aldehyde group moiety of glyoxylic acid or the like, and an amino group moiety of an oxyamino group or the like; and the like. Here, in the above-mentioned Schiff base formation reaction, the formed Schiff base group may be reduced with a proper reducing agent.

The above-mentioned method of reaction of formation of a thiazolidine ring includes, for example, a method including the step of reacting cysteine in which an amino group and a thiol group remain unreacted, and an aldehyde group; and the like.

The reaction of introduction by a non-covalent bond includes complex formation reactions (for example, a His-Tag formation reaction utilizing a ternary complex formation of nickel-trinitriloacetic acid complex and an oligohistidine, a gold-thiol coupling reaction, and the like); biologically recognizable reactions (for example, a sugar chain-lectin reaction, avidin-biotin reaction, an antigen-antibody reaction, and the like); and the like.

The reaction for introduction can be carried out by providing a combination of a functional group for binding ligand molecules and a functional group for binding to a spacer in the form which is capable of binding to each other in the above-mentioned reaction embodiment. The combination as described above can be appropriately selected referring to those given above. Here, the reaction for introduction as described above may be initiated by a different factor such as light, heat, vibration, time period, or deprotection.

As the functional group for binding to a spacer, a reactive amino acid residue, a reactive site contained in a saccharide originally contained in the ligand molecule, or the like may be used, or a functional group for binding to a spacer intentionally introduced into the ligand molecule may be used. The method of introducing a functional group for binding to a spacer into a ligand molecule includes a method of chemically modifying a ligand molecule by using a bifunctional reagent containing a functional group for binding to a spacer, and containing a functional group for binding ligand molecules in the same molecule. One method of chemically modifying a ligand molecule containing a peptide includes the steps of synthesizing ligand molecules, and thereafter coupling the ligand molecules to a bifunctional reagent utilizing reactivity of an amino acid residue, or the like, and a known method described in, for example, one edited by Toru Komano, Kensuke Shimura, Kenzo Nakamura, Michinori Nakamura, and Nobuyuki Yamazaki, and authored by Motonori Ohno, Yuichi Kaneoka, Fumio Sakiyama, and Hiroshi Maeda, "Tanpakushitu no Kagakushushoku <Jo> (Chemical Modification of Proteins <volume one>)," Seibutsukagaku Jikken Ho(Experimental Methods in Biological Chemistry) 12, Japan Scientific Societies Press (1981), or the like is used. In addition, a functional group can be freely introduced into an N-terminal or an amino acid residue or the like of the ligand molecule at the stage of chemically synthesizing the ligand molecule by using a known method described in, for example, MW. Pennington, BM. Dunn Ed., "Peptide Synthesis Protocols", Methods in Molecular Biology, 35, Humana Press (1994) or the like. The same can be also said for a ligand molecule containing a saccharide, and a known method described in, for example, one edited by Tousa Kougaku Henshu Iinkai (The Editorial Committee for Sugar Chain Engineering of Japan), "Tousa Kougaku (Sugar Chain Engineering)," Industrial Research Center of Japan, Baiotekunoroji Johou Sentah (Biotechnology Information Center) (1992), or the like can be used.

As a method of introducing a functional group for binding ligand molecules into a single-stranded nucleic acid, in other words, DNA, RNA, PNA, or a derivative thereof, position-specifically, a method described in, for example, a publication edited by The Society of Polymer Science, Japan/Research Group on Polymers and Biosciences, "Koubunshi Kagaku to Kakusan no Kino Dezain (Polymer Chemistry and Functional Designs of Nucleic Acids)"; a publication of Baio Koubunshi Kenkyuho (Research Methods for Polymers and Biosciences) 6, Japan Scientific Societies Press (1996); Y. Ito, E. Fukusaki, J. Mol. Catal. B: Enzymatic, 28, 155-166 (2004); reference publications listed in the publications; and the like can be utilized. Exemplifications include a chemically modified product obtained by introducing a functional group for binding ligand molecules into a phosphate site, or a main chain containing ribose, deoxyribose, or the like (2-aminoethyl-glycine main chain in the peptide nucleic acid), or a moiety of a nucleic acid base, or the like; and a chemically modified product obtained by introducing a functional group for binding ligand molecules into a 5'-terminal and/or a 3'-terminal (a chemically modified product obtained by introducing a functional group into an N-terminal and/or a C-terminal in the peptide nucleic acid). As described above, the methods are not limited to those exemplified above, so long as the position of the functional group for binding ligand molecules can be specified in the nucleic acid spacer.

A linker may be contained between a nucleic acid spacer and a functional group for binding ligand molecules, and/or between ligand molecules and a functional group for binding to a spacer. The purpose of the linker is to slightly set apart a distance between the nucleic acid spacer and the ligand molecules, whereby exhibiting a function of reducing steric hindrance or the like in the reaction between the nucleic acid spacer and the ligand molecules, thereby quickly progressing the reaction of introduction; and a function of not encumbering a binding reaction of a cell surface receptor and ligand molecules in a case where the ligand construct having a nanostructure of the present invention is contacted with cells; and the like. The materials and the like of the linker are not particularly limited, so long as the linker has a length of usually from about 0.1 to about 5 nm, which may be, for example, an alkyl chain, a peptide chain, an oxyethylene chain, an oxypropylene chain, or the like, or a mixed structure thereof. Here, the linker is not limited to the structure given above, and an optimal one is selected depending upon the purposes. The linker may be introduced into a nucleic acid spacer and/or ligand molecules as, for example, a part of a functional group for binding ligand molecules or a functional group for binding to a spacer.

In a case where a nucleic acid spacer is constituted by two or more strands of nucleic acid strands using a nucleic acid as the spacer, two methods can be exemplified as a step of binding ligand molecules to a nucleic acid spacer. In other words, the methods are a method including the step of previously binding ligand molecules via a functional group at the stage of a single-stranded nucleic acid having a functional group for binding ligand molecules (which may be hereinafter referred to as "pre-modification method"); and a method including the step of binding ligand molecules to a perfected nucleic acid spacer having a functional group for binding ligand molecules (which may be hereinafter to as "post-modification method").

The pre-modification method of ligand molecules will be described in more detail. The method is a method including the steps of previously binding ligand molecules at the stage of a single-stranded nucleic acid containing a functional group for binding ligand molecules, and subsequently further combining the ligand molecules with one or more kinds of single-stranded nucleic acids (the nucleic acids may or may not contain a functional group for binding ligand molecules), thereby perfecting a nucleic acid spacer introduced into the ligand molecules position-specifically.

The after-modification method of ligand molecule will be described in more detail. The method is a method including the steps of further combining a single-stranded nucleic acid containing a functional group for binding ligand molecules with one or more kinds of single-stranded nucleic acids (the nucleic acids may or may not contain a functional group for binding ligands), thereby perfecting a nucleic acid spacer (not yet introduced into the ligand molecules at this point); and subsequently introducing thereinto ligand molecules having a functional group for binding to a spacer. Either of the pre-modification method or the after-modification method mentioned above can be selected as occasion demands, and the pre-modification method is more preferably employed in consideration of the facilitation of the preparation.

The ligand construct having a nanostructure of the present invention is defined by the structural features as described above. It is preferable that deoxyribonucleic acid is utilized as a nucleic acid spacer taking into consideration facilitation in the synthesis of a long-chained spacer exceeding 30mers of nucleotides, and the like. Especially, a double helical DNA strand has a length of 3.4 nm in 10 nucleotides, showing a definite structure; therefore, it is desired that a double helical DNA strand (which may be hereinafter also referred to as "dsDNA") is especially preferably used as a nucleic acid spacer.

The structure of the ligand construct having a nanostructure of the present invention will be explained more concretely hereinbelow. A preferred position of a nucleic acid spacer containing a dsDNA into which ligand molecules are introduced includes terminal sites of dsDNA spacer, such as 5'-terminal (there are a total of two positions, if bound to a case where there are no midway branching, or the like), and 3'-terminal (there are a total of two positions, if bound to a case where there are no midway branching, or the like); and arbitrary positions in the midway of a dsDNA spacer, such as nucleic acid base sites. Cases where two ligand molecules are contained will be listed as follows: A molecule in which each of ligand molecules is bound to two 5'-terminals of the dsDNA chain, a molecule in which each of the ligand molecules is bound to two 3'-terminals of the dsDNA chain, or a molecule of a combination thereof (for example, a molecule in which a ligand molecule is bound to each of 5'-terminal and 3'-terminal, and the like). In a case where three or more ligand molecules are introduced, a vacant terminal can be utilized. Those given above are mere exemplifications, and the positions at which the ligands are introduced is not particularly limited, and can be changed in accordance with the purposes.

In order to introduce ligand molecules according to the pre-modification method, a functional group for binding ligand molecules is introduced into a single-stranded DNA (which may be hereinafter also referred to as ssDNA), which is a raw material for a nucleic acid spacer, at the stage of the chemical synthesis of DNA. As the functional group for binding ligand molecules, various kinds can be selected as mentioned above, without being limited. Among those exemplified above, it is preferable to introduce a thiol group or an amino group, from the viewpoint of exhibiting a high reaction selectivity.

It is preferable that a functional group for binding to a spacer having binding activity with a thiol group or an amino group is introduced into the ligand molecules in advance. The above-mentioned functional group for binding to a nucleic acid spacer is not particularly limited, and the functional group includes, for example, a maleimide group, an acrylic ester group, an acrylamide group, a vinylsulfone group, or the like for a Michael addition reaction with a thiol group; a haloacetyl group, an epoxy group, an aziridine group, or the like for a thioether formation reaction with a thiol group. Among them, a maleimide group and a haloacetyl group can be exemplified as preferred functional groups for binding to a spacer, from the viewpoint of easiness in introduction into the ligand molecules and stability.

A case where a synthetic peptide molecule is used as a ligand molecule will be described in further detail. Upon synthesizing a synthetic peptide molecule, a functional group for binding to a spacer can be easily introduced into ligand molecules by treating an amino group side chain such as a lysine residue, an N-terminal amino group, or the like with a maleimide derivative (for example, N-hydroxysuccinimide maleiimideacetic acid ester, N-hydroxysuccinimide 4-maleidobutanoic acid ester, or the like), or a haloacetyl derivative (for example, N-hydroxysuccinimide bromoacetic acid ester, N-hydroxysuccinimide 6-(iodoacetamide)caproic acid ester, iodoacetic anhydride, or the like), which is a bifunctional reagent having a carboxylic acid, an acid chloride, an active ester group, or the like.

By using the ssDNA and the ligand molecules as described above, ssDNA obtained by introduction of the ligand molecules (the ssDNA into which the ligand molecules are introduced may be hereinafter simply referred to as a "conjugate") can be prepared. The reaction conditions, the purification method, and the like are not particularly limited, and a known method can be utilized. Using a single-stranded DNA to which the ligand molecules obtained as described above are introduced as a raw material, the ligand construct of the present invention is obtained by carrying out hybridization with other ssDNA (this ssDNA may not be necessarily bound to a ligand molecule), or ligation, as occasion demands, with dsDNA (this dsDNA may not be necessarily bound to a ligand molecule). In addition, the ligand construct of the present invention can also be prepared by using a polymerase chain reaction with the conjugate as a primer.

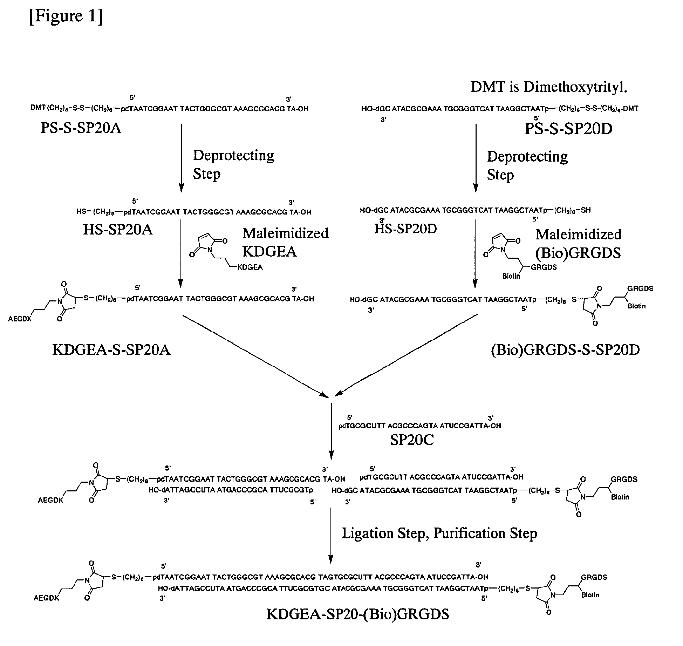

Here, in a case where the above-mentioned nucleic acid spacer contains a plural nucleic acids, at least two, identical or different, ligand molecules may be each bound to identical or different nucleic acids, respectively. For example, in a case where the nucleic acid spacer contains a nucleic acid forming a double-stranded chain, and two ligand molecules are each bound to a 5'-terminal or a 3'-terminal of the nucleic acid, there exist two 5'-terminals and 3'-terminals in the nucleic acid spacer. In this case, if ligand molecules are bound to both of the terminals of the identical nucleic acid, the ligand molecules are bound to the identical nucleic acid; and if one 5'-terminal of the nucleic acid is bound to one ligand molecule, and the other 5'-terminal of the nucleic acid is bound to another ligand molecule, the ligand molecules are bound to different nucleic acids (see, for example, Figure 1).

The ligand construct as described above is one embodiment of the present invention, and is not limited thereto. As already mentioned above, it is obvious that the method for introducing a functional group for binding ligand molecules into the ssDNA, sites, and the kinds, the method for introducing a functional group for binding to a spacer into the ligand molecules, sites, and the kinds of the functional groups are selected according to their purposes. In addition, as already mentioned above, it is probably obvious using the above technical idea that the ligand construct of the present invention including a nucleic acid spacer having a structure that is not only a simple double helical structure, but also various complicated structures, for example, a branched structure or a three-dimensional structure, the structure in which nucleic acid spacers are multiply coupled two-dimensionally or three-dimensionally, or the like, can be prepared.

In a case where a polyamino acid is used as a spacer, similarly to the case of the nucleic acid, a reactive group originally contained in the polyamino acid, an intentionally introduced reactive group, or the like may be used as a functional group for binding ligand molecules, or a functional group for binding ligand molecules may be introduced into the above-mentioned reactive group by chemical modification and used.

A method for introducing a functional group for binding ligand molecules into a spacer containing a polyamino acid includes a method of chemically modifying a spacer containing a polyamino acid using a bifunctional reagent containing a functional group for binding ligand molecules, and containing a functional group for binding to a spacer molecule in the same molecule (which may be hereinafter simply referred to as a reagent containing a functional group for binding ligand molecules).

One method of chemically modifying a spacer containing a reactive amino acid residue includes the step of synthesizing a spacer molecule, and thereafter binding thereto a reagent containing a functional group for binding ligand molecules utilizing a reactivity of an amino acid residue, or the like. For example, a known method as described, for example, in one edited by Toru Komano, Kensuke Shimura, Kenzo Nakamura, Michinori Nakamura, and Nobuyuki Yamazaki, and authored by Motonori Ohno, Yuichi Kaneoka, Fumio Sakiyama, and Hiroshi Maeda, "Tanpakushitu no Kagakushushoku <Jo> (Chemical Modification of Proteins <volume one>)," Seibutsukagaku Jikken Ho(Experimental Methods in Biological Chemistry) 12, Japan Scientific Societies Press (1981), or the like is used. In addition, a reagent containing a functional group for binding ligand molecules can be freely introduced into an N-terminal, amino acid residue or the like at the stage of chemically synthesizing a spacer by using a known method described in, for example, MW. Pennington, BM. Dunn Ed., "Peptide Synthesis Protocols", Methods in Molecular Biology, 35, Humana Press (1994) or the like.

By reacting a spacer containing a polyamino acid into which a functional group for binding ligands is introduced with ligand molecules having a functional group for binding to a spacer as described above, a ligand construct in which a polyamino acid serves as a spacer can be obtained.

A case where PEG or PPG is used among the synthetic polymers as a spacer will be described. Similarly, in this case, as the functional group for binding ligand molecules, reactive groups at both terminals of the polymer chain originally contained in the PEG or PPG chain, both terminal reactive groups that are intentionally introduced, or the like may be used, or functional groups for binding ligand molecules may be introduced into both terminals by chemical modification, and used.

In other words, a linear PEG or PPG chain in which a terminal functional group can be used as a functional group for binding ligand molecules is exemplified by those of which two terminal reactive groups are hydroxyl groups, amino groups, or carboxyl groups, or those of which one terminal reactive group is an amino group, and the other terminal reactive group is a carboxyl group.

In addition, a method of binding a functional group for binding ligand molecules to the reactive group mentioned above, using a reagent containing a functional group for binding ligand molecules can be adopted. By treating a spacer containing PEG or PPG into which a functional group for binding ligand molecules is introduced with a ligand molecule having a functional group for binding a spacer as described above, a ligand construct containing PEG or PPG as a spacer can be obtained.

The ligand construct having a nanostructure of the present invention prepared in the manner described above may be used alone, or in a mixture of two or more kinds. The ligand construct that is optimal for each of the cells is selected.

The ligand construct of the present invention contains, in addition to ligand molecules and a spacer, one or more functional groups for binding substrates, that is used for immobilization to the substrates. By having such a constitution, it is made possible to easily immobilize the ligand construct to a substrate so long as a functional group capable of binding a functional group for binding substrates (which may be hereinafter simply referred to as "functional group for binding a ligand construct") is contained on a substrate surface, without being dependent on the properties of the substrate.

An immobilization reaction of a ligand construct to a substrate (in other words, a combination of a functional group for binding substrates and a functional group for binding a ligand construct) is not particularly limited, so long as the ligand construct and the substrate are stably maintained during the reaction, and the reaction of introduction can be carried out specifically to the intended sites themselves. The combination may be a combination for forming a covalent bond, or a combination for forming a non-covalent bond.

Examples of the reaction for forming a covalent bond include the same ones as those reactions mentioned above, and the reactions include, for example, amide bond formation reaction, Michael addition reaction, thioether formation reaction, Diels-Alder cyclization addition reaction, Schiff base formation reaction, reaction of formation of thiazolidine ring, and the like. In these reactions, a reactive amino acid residue originally contained in a ligand molecule, a linker, and a spacer, a reactive site contained in a saccharide or the like may be used as a functional group for binding substrates, or a functional group for binding substrates may be intentionally introduced into a ligand molecule, a linker, and a spacer and used as a functional group for binding substrates. On the other hand, examples of forming a non-covalent bond include, for example, avidin(streptavidin)-biotin, an antigen-antibody reaction, a sugar chain-lectin reaction, and the like. Among them, in order to immobilize a molecule having a relatively high molecular weight such as the ligand construct of the present invention to a substrate, it is preferable to select a reaction having a relatively fast reaction rate, and the reaction includes, for example, a method employing an immobilization reaction by a non-covalent bond. A preferred immobilization reaction by a non-covalent bond includes avidin(streptavidin)-biotin, an antigen-antibody reaction, a sugar chain-lectin reaction, and the like, among which a more preferred reaction is exemplified by a method employing biologically recognizable reaction, especially a coupling reaction of avidin(streptavidin)-biotin, from the viewpoint of immobilization efficiency.

Avidin is a basic protein capable of specifically binding to biotin existing in albumen, that is a tetramer having a molecular weight of roughly 68,000 or so. Streptavidin is a protein derived from a microbial culture medium of *Streptomyces avidinii*, that has a high binding ability to biotin, which is a kind of vitamin, in the same manner as in avidin. The avidin and the streptavidin will be hereinafter collectively referred to as avidin.

Either avidin or biotin may be introduced into a ligand construct as a functional group for binding substrates, and it is preferable that biotin is introduced into a ligand construct, from the viewpoint of facilitation in the preparation of the ligand construct. A site into which biotin is introduced may be any one of a ligand molecule site, a linker, and a spacer site of the ligand construct, and the number of introduction may be one or more.

In addition, in the present invention, for example, a biotin derivative, such as a molecule containing a carboxyl group active ester activated with an amino group, a carboxyl group, an N-hydroxysuccinimide or the like, a thiol group, a haloacetyl group, an $\alpha,\beta$-unsaturated carbonyl group such as a maleimide, or a hydrazine group, and coupled to a biotin group via a linker, can be also used as a functional group for binding substrates or a functional group for binding a ligand construct. Concrete examples of the biotin derivatives include biotin derivative which can be purchased from Pierce Biotechnology, and the trade names can be exemplified by NHS-Biotin, Sulfo-NHS-Biotin, NHS-LC-Biotin, Sulfo-NHS-LC-Biotin, Sulfo-NHS-LC-LC-Biotin, Sulfo-NHS-SS-Biotin, NHS-PEO$_4$-Biotin, NHS-LC-LC-Biotin, PFP-Biotin, TFP-PEO-Biotin, PEO-Maleimide Activated Biotin, Biotin-BMCC, PEO-Iodoacetyl Biotin, Iodoacetyl-LC-Biotin, Biotin-HPDP, 5-(Biotinamido)pentylamine, Biotin PEO-Amine, Biotin PEO-LC-Amine, Biocytin Hydrazide, Biotin Hydrazide, Biotin-LC-Hydrazide, or the like.

In addition, an avidin (streptavidin) derivative such as a derivative of which amino acid residue is chemically modified (which can be modified by a method described, for example, in one edited by Toru Komano, Kensuke Shimura, Kenzo Nakamura, Michinori Nakamura, Nobuyuki Yamazaki, authored by Motonori Ohno, Yuichi Kaneoka, Fumio Sakiyama, and Hiroshi Maeda, "Tanpakushitu no Kagakushushoku <Jo> (Chemical Modification of Proteins <volume one>)," Seibutsukagaku Jikken Ho(Experimental Methods in Biological Chemistry) 12, Japan Scientific Societies Press (1981), or the like), or a derivative in which amino acids are substituted site-specifically can be also used as a functional group for binding substrates or a functional group for binding a ligand construct.

A method of introducing a functional group for binding substrates into a ligand molecule site or a spacer site include a method of modification by forming a covalent bond during synthesis, or after the synthesis of ligand molecules and a spacer, using a bifunctional reagent containing a functional group for binding substrates, and containing in the same molecule a functional group binding ligand molecules and/or binding to a spacer site (which may be hereinafter simply referred to as a reagent containing a functional group for binding substrates).

One of the method of modifying a functional group for binding substrates to ligand molecules containing a peptide, or a spacer containing a polyamino acid includes the steps of synthesizing ligand molecules or a spacer, or coupling ligand molecules with a spacer (or a raw material thereof), and subsequently binding a reagent containing a functional group for binding substrates to a reactive amino acid residue originally existing in, or intentionally introduced into, the ligand molecules or the spacer (or a raw material thereof), and a known method described in, for example, one edited by Toru Komano, Kensuke Shimura, Kenzo Nakamura, Michinori Nakamura, authored by Nobuyuki Yamazaki, Motonori Ohno, Yuichi Kaneoka, Fumio Sakiyama, and Hiroshi Maeda, "Tanpakushitu no Kagakushushoku <Jo> (Chemical Modification of Proteins <volume one>)," Seibutsukagaku Jikken Ho(Experimental Methods in Biological Chemistry) 12, Japan Scientific Societies Press (1981), or the like can be used. In addition, a bifunctional reagent containing a functional group for binding substrates can be arbitrarily introduced into an N-terminal, reactive amino acid residue or the like at the stage of chemically synthesizing ligand molecules by using a known method described in, for example, MW. Pennington, BM. Dunn Ed., "Peptide Synthesis Protocols", Methods in Molecular Biology, 35, Humana Press (1994) or the like.

Similar to the case of ligand molecules containing a saccharide, a known method described in, for example, one edited by Tousa Kougaku Henshu Iinkai (The Editorial Committee for Sugar Chain Engineering of Japan), "Tousa Kougaku (Sugar Chain Engineering)," Industrial Research Center of Japan, Baiotekunoroji Johou Sentah (Biotechnology Information Center) (1992), or the like is used, whereby a reagent containing a functional group for binding substrates can be arbitrarily introduced into a sugar chain.

In the case where a spacer is PEG or PPG, a functional group for binding substrates can be introduced into a terminal part of the spacer. As already explained above, a terminal reactive group of the spacer containing PEG or PPG may be also used as a functional group for binding ligand molecules. Therefore, upon introduction of an active group for binding substrates into a terminal reactive group of the spacer containing PEG or PPG, it is preferable that a reagent containing an active group for binding substrates further contains a functional group for binding ligands.

Similarly, in the case where a nucleic acid is used as a spacer, a reagent containing a functional group for binding substrates can be bound to a reactive group or the like originally contained in the nucleic acid, a reactive group intentionally introduced thereinto, or the like, and used.

In addition, a method for introducing a functional group for binding substrates into a ligand construct via a linker includes a method of modification by a reaction of forming a covalent bond during synthesis of a linker site, or after the synthesis, applying a bifunctional reagent containing a functional group for binding substrates, and containing in the same molecule a functional group for binding ligand molecules and/or that binding to a spacer site (which may be hereinafter simply referred to as a reagent containing a functional group for binding substrates) to a reactive functional group originally existing in the linker site, or intentionally introduced thereinto.

In the case where a ligand construct of the present invention is used in the regulation of adherent cell functions, it is preferable that a ligand construct is used in the state that the ligand construct is immobilized to a substrate, the ligand construct being usually immobilized to a substrate made of biodegradable or non-biodegradable material, and used. Therefore, a cell culture substrate of the present invention containing the ligand construct and a substrate moiety, wherein the ligand construct is immobilized to the substrate moiety via a functional group for binding substrates, is also provided as one embodiment of the present invention. In a case, for example, where the ligand construct is immobilized to a substrate made of a biodegradable material, it is assumed that the transplanted material does not remain after the material is used in the transplantation into a living body. In other cases, the material is not particularly necessarily a biodegradable material, and can be selected depending upon its purposes.

Among the substrates to which a ligand construct is immobilized, biodegradable materials include polyesters (for example, polylactic acid, polyglycolic acid, a copolymer of lactic acid and glycolic acid, a copolymer of lactic acid or glycolic acid and polyethylene glycol, poly($\varepsilon$-caprolactone), poly(3-hydroxybutyrate), poly(p-dioxanone), polypropylene fumarate, and the like); polyorthoesters (for example, polyol/diketene acetal addition polymers, and the like); polyanhydrides (for example, poly(sebacic acid anhydride), poly(carboxybiscarbioxyphenoxyphenoxyhexane), poly[bis(p-carboxyphenoxy)methane], copolymers of monomers contained in the above polymers, and the like), polyamino acids; polyphosphazenes; proteins (for example, serum albumin, collagen, silk fibroin, fibroin, avidin, streptavidin, and the like); polysaccharides (for example, alginic acid, starch, hyaluronic acid, dextran, cellulose, and the like and derivatives thereof); and the like.

Among the substrates to which a ligand construct is immobilized, as the non-biodegradable materials, general polymer materials and inorganic materials are used. The addition polymerization polymer includes, for example, polyacrylic acid and a derivative thereof, polyethylene, polypropylene, polystyrene, polyethylene glycol, polyacrylamide, polyvinyl alcohol derivatives, ethylene-vinyl alcohol copolymers and derivatives thereof, polytetrafluoroethylene, and the like. The polycondensation polymer is, for example, polyesters such as polyethylene terephthalate; polyamides such as nylon-6,6; polyimides; polyurethanes; and the like. The inorganic material includes metals such as titanium/titanium oxide, and gold; hydroxyapatite; glass; silicon; and the like. The materials are, however, not limited to those exemplified, and an optimal material is selected depending upon its purpose. In addition, the above material may be subjected to various modification treatments including, for example, plasma treatment, ion implantation treatment, rubbing treatment, chemical oxidation treatment, hydrolysis treatment, or the like.

The above substrate may be provided with a coating on its surface. The coating layer includes, for example, a grafted polymer layer; a monomolecular film layer (for example, a self-assembling monomolecular film or the like described in RS. Kane et al., Biomaterials, 20, 2363-2376 (1999), or the like can be utilized); a hydrogel layer (for example, a layer coated with a hydrogel as described in one edited by Osada and Kajihara, "Geru Handobukku (Gel Handbook)," NTN, 1997) or the like); a protein layer (for example, a layer coated with a polyamino acid, a protein, or the like); an adsorbent polymer layer (for example, a layer coated with a hydrophilic polymer, such as polylysine, pluronic (a block polymer of polyethylene oxide and polypropylene oxide), poly(2-methoxyethyl acrylate), or a polysaccharide); and the like.

As the substrates and modification treatments thereof and the coating layer exemplified above, most appropriate ones are selected depending upon the cells to be intended. It is necessary to at least contain a functional group for binding a ligand construct, in order to immobilize the ligand construct having a functional group for binding substrates of the present invention.

As a method of including a functional group for binding a ligand construct in the substrate moiety of the cell culture substrate of the present invention, a reactive group of a substrate or a coating layer, for example, a residual carboxyl group of a polyester, a residual amino group of a polyamide or a protein, a reactive amino acid residue of a polyamino acid group, a hydroxyl group of a polysaccharide, an amino group, or a carboxyl group, or the like may be utilized as the functional group for binding a ligand construct. A bifunctional reagent containing a functional group for binding a ligand construct and a functional group for binding substrates may be bound to a substrate, using a reaction for forming a covalent bond.

In order to introduce a functional group for binding a ligand construct into a substrate, it is preferable to use avidin, from the viewpoint of immobilization efficiency or the like. Since avidin is a protein, avidin can be allowed to be contained on the substrate surface by binding a reactive amino acid residue contained in avidin with a functional group of a substrate itself or a coating layer utilizing an amide formation reaction or the like. In addition, a protein such as avidin has been well known to physically adsorb to a hydrophobic surface, such as polystyrene, so that the protein can be immobilized to a substrate surface by using the method as described above to allow the protein to be contained in the substrate surface.

If a protein such as avidin is bound to a substrate utilizing an amide formation reaction or the like, or physically adsorbed to a substrate surface, the structure may undergo changes, so that the binding activity may be lowered than that inherently owned by a protein. In the present invention, in order to allow avidin to contain on the surface, a method including the steps of first binding biotin to a substrate, and then binding avidin to the substrate may be employed. Since one molecule of avidin can be bound to four biotin molecules; therefore, even if a part of the biotins is used in the surface immobilization of avidin, the remaining binding sites can be used in the immobilization of the ligand construct having a functional group for binding substrates.

Upon the immobilization of biotin to a substrate surface, biotin can be introduced into a reactive group of a substrate by a reaction of forming a covalent bond, or a reaction of forming a non-covalent bond. Taking stability of the bond or the like into consideration, the covalent bond is preferred as an immobilization reaction. The covalent bond is obtained by reacting with a substrate a bifunctional reagent containing a functional group capable of binding to biotin and a substrate (which may be hereinafter simply referred to as a reagent containing biotin).

The reagent containing biotin will be even more concretely exemplified. The reagent containing biotin includes, for example, one containing an amino group, a carboxyl group, a carboxyl group active ester activated with an N-hydroxysuccinimide or the like, a thiol group, a haloacetyl group, an $\alpha,\beta$-unsaturated carbonyl group such as a maleimide group, or a hydrazine group, being coupled to a biotin group via a linker. Even more specific examples include biotin derivatives which can be purchased from Pierce Biotechnology. As the trade names, NHS-Biotin, Sulfo-NHS-Biotin, NHS-LC-Biotin, Sulfo-NHS-LC-Biotin, Sulfo-NHS-LC-LC-Biotin, Sulfo-NHS-SS-Biotin, NHS-PEO$_4$-Biotin, NHS-LC-LC-Biotin, PFP-Biotin, TFP-PEO-Biotin, PEO-Maleimide Activated Biotin, Biotin-BMCC, PEO-Iodoacetyl Biotin, Iodoacetyl-LC-Biotin, Biotin-HPDP, 5-(Biotinamido)pentylamine, Biotin PEO-Amine, Biotin PEO-LC-Amine, Biocytin Hydrazide, Biotin Hydrazide, Biotin-LC-Hydrazide or the like can be used as a reagent containing biotin.

In a case where a reagent containing biotin contains an amino group, a carboxyl group or an aldehyde group is preferred as a reactive group of a substrate, and an amide bond formation reaction or a Schiff base formation reaction can be respectively used as a means of introduction of the reagent into the substrate. In a case where a reagent containing biotin contains a carboxyl group or a carboxyl group active ester, an amino group is preferred as a reactive group of a substrate, and an amide-bond formation reaction can be used as the means of introduction mentioned above. In a case where a reagent containing biotin contains a thiol group, a haloacetyl group or an epoxy group is preferred as a reactive group of a substrate, and a thioether formation reaction can be used as the means of introduction mentioned above. In a case where a reagent containing biotin contains a haloacetyl group or an $\alpha,\beta$-unsaturated carbonyl group as a reactive group of a material or a coating layer, a thiol group is preferred, and a thioether formation reaction can be used as the means of introduction mentioned above.

By treating a substrate into which biotin is introduced in the manner as described above with avidin in a water-dissolved state for a given period of time, to bind the substrate and avidin, a substrate containing avidin can be prepared. Examples of concrete reaction conditions include conditions that a temperature is preferably 60°C or less, and more preferably 40°C or less, that a pH is preferably within the range of from 3 to 11, and more preferably within the range of from 4 to 9, for 1 hour or more. By controlling the concentration of an aqueous avidin solution to be added to a substrate into which biotin is introduced, the surface density of the avidin can be freely changed. Avidin has a surface density of preferably 0.1 fmol/cm$^2$ or more, for the purpose of securing immobilization density of a ligand construct, and the avidin has a surface density of even more preferably 1 fmol/cm$^2$ or more, taking the lowering of the binding activity to biotin upon immobilization of avidin into consideration. On the other hand, avidin preferably has a surface density of 15 pmol/cm$^2$

or so or less, taking the size of avidin or the like into consideration. Here, the density of the immobilized avidin may be changed, depending upon the surface roughness, the thickness of the coating layer, and the like.

The substrate to which a ligand construct having a nanostructure is immobilized may be made of, for example, woven fabric, nonwoven fabric, flocculent product, filament, hollow fiber, porous material, spherical material, cylindrical material, film, plate, and composite structures thereof (for example, a multi-well plate or the like can be exemplified), and the size of filament, sphere, pore, cylinder or the like contained in each form may be a nanometer size. Also, regarding each of the substrates, a fine processing in the order of nanometer size or more may be provided by a known method, and the fine processing includes, for example, a circuit forming a microcurrent path as described in K. Sato, A. Hibara, M. Tokeshi, H. Hisamoto, T. Kitamori, Analytical Sciences, 19, 15-22(2003); a plate forming a cell adherent region and non-adherent region in the order of micrometer size as described in Non-Patent Publication 8 or the like. The ligand construct of the present invention may be immobilized by drawing a pattern of a dot form, a line form, or a combination thereof, having the size of micrometers to nanometers to the material according to the fine processing method exemplified above, including, for example, inkjet lithography method, optical lithography method, micro-contact printing method, dipping nanolithography or the like. These can be selected as occasion demands.

The use embodiment of the ligand construct having a nanostructure of the present invention is preferably an embodiment in which the ligand construct is immobilized to the substrate as described above and used. As a method for immobilization, a known method by a covalent bond or a non-covalent bond can be properly utilized, depending upon the kinds of the functional groups for binding substrates owned by the above-mentioned ligand construct, and the immobilization by a non-covalent bond is preferably used, judging from the facilitation of the immobilization. In the immobilization by a non-covalent bond, for example, the method as described above, or the like can be utilized.

Upon the immobilization of the ligand construct having a nanostructure of the present invention to a substrate, the surface density of the ligand construct of the present invention can be freely selected depending upon the cells of interest, and is not particularly limited. However, it is preferable that the surface density of the ligand construct is within the range that an average distance between the ligand constructs as defined by the following formula:

$$\text{Average Distance} = 1000/(6.02 \times \text{Surface Density})^{1/2}$$

is not shorter than the length of a spacer contained in the ligand construct of the present invention. The unit of an average distance between the ligand constructs is nanometer (nm), and the unit of the surface density of the ligand molecules is femtomol per 1 square centimeter (fmol/cm$^2$). By the above calculation formula, the maximum surface density can be selected for each spacer depending upon the lengths of various spacers, and the preferred surface density of the ligand construct is 166 pmol/cm$^2$ (166 $\times$ 10$^3$ fmol/cm$^2$) or less, more preferably 6.6 pmol/cm$^2$ (6.6 $\times$ 10$^3$ fmol/cm$^2$) or less, and even more preferably 2.0 pmol/cm$^2$ (2.0 $\times$ 10$^3$ fmol/cm$^2$) or less, taking into consideration the preferred range of the length of the spacer already described. The lower limit of the surface density is not particularly limited, and the lower limit is preferably about 0.1 fmol/cm$^2$ or more, a minimum density at which the cells can adhere, or more, as described in, for example, SP. Massia, JA. Hubbell, J. Cell Biol., 114(5), 1089-1100(1991) or the like.

The ligand construct of the present invention and a substrate immobilized therewith can be sterilized as occasion demands. The method of sterilization is not particularly limited, and any one of methods can be employed according to their applications, and the method includes, for example, autoclave sterilization, ethylene oxide gas sterilization, γ-ray sterilization, electron beam sterilization, sterilization by filtration, and the like. A method combining the above methods, for example, a method of immobilizing under sterile conditions a ligand construct sterilized by filtration to a material or a substrate previously sterilized, thereby preparing the ligand construct of the present invention and materials immobilized therewith can be performed, and an optimal method of sterilization can be selected as occasion demands.

The ligand construct having a nanostructure of the present invention can achieve its object by the interaction with cells in the state of a solution or in a state of immobilization to a substrate, and culture of the cells. In order for the cells to exhibit their functions, a medium is necessary in addition to the ligand construct of the present invention. Although the media are subject to change depending upon the kinds of the cells used, and the like, the medium includes MEM medium, BME medium, DME medium, α-MEM medium, IMEM medium, ES medium, DM-160 medium, Fisher medium, F12 medium, WE medium, RPMI medium, and mixtures thereof (for example, one published by Asakura Shoten, edited by Nihon Soshiki Baiyo Gakkai, "Soshiki Baiyo no Gijutsu (Techniques of Tissue Culture)," Third Edition, page 581), and those in which sera components (for example, bovine sera, and the like) and the like are added to these media. The culture of the cells may be at least carried out in the presence of the ligand construct of the present invention (for example, those prepared by adding the ligand construct of the present invention in the state of a solution to these media may be used), and it is preferred that the cells are cultured using the cell culture substrate of the present invention, from the viewpoint of effectively accomplishing the regulation of the cell functions. Therefore, in another embodiments, the present invention provides cells obtained by culturing cells in the presence of the ligand construct of the present invention, and a method for preparing cells including the steps of culturing cells in the presence of the ligand construct of the present invention. Here, the concrete methods for cell culture may be referred to above-mentioned text references, and the like.

The term "cell function" as used to herein refers to an ability of cells showing the state of the cells according to their purposes, including, for example, adhesion, proliferation, differentiation, undifferentiation, survival and/or cell death, or the like. The ligand construct having a nanostructure of the present invention can be utilized as a material for regulating a function of the cells which serves as a core, in an instrument for evaluating various substances using the cells. In addition, the ligand construct can be utilized as a scaffold component of the like in cell (regenerative) therapy. Therefore, since the cells used and the required functions vary depending upon a wide variety of applications as described above, especially cells and functions to be regulated thereof may be determined according to their purposes and not particularly limited.

EXAMPLES

The present invention will be specifically described hereinbelow by Examples and the like, without intending to limit the present invention thereto. The measurement methods or evaluation methods used in the following Examples and the like are collectively shown below. Incidentally, in the following description, "%" means "% by volume," unless specified otherwise.

(Analysis of Peptide According to Reversed Phase High Performance Liquid Chromatography (HPLC))

A peptide was analyzed according to reversed phase HPLC under the following conditions. TSKgel ODS-80TM manufactured by Tosoh Corporation was used as a column. The flow rate of an eluent upon elution was 1.0 mL/min, and the detected wavelength was 210 nm. In condition a, using a 5% aqueous acetonitrile solution containing 0.05% trifluoroacetic acid (which may be hereinafter abbreviated as TFA) as eluent A, and a 30% aqueous acetonitrile solution containing 0.05% TFA as eluent B, the elution was carried out with a linear gradient from eluent A to eluent B for 20 minutes. Also, in condition b, using a 5% aqueous acetonitrile solution containing 0.05% trifluoroacetic acid as eluent A, and a 72.5% aqueous acetonitrile solution containing 0.05% TFA as eluent B, the elution was carried out with a linear gradient from eluent A to eluent B for 45 minutes.

(Purification of Peptide According to Reversed Phase HPLC)

A peptide was purified according to reversed phase HPLC under the following conditions. PREP-ODS manufactured by Shimadzu Corporation was used as a column. The flow rate of an eluent upon elution was 10 mL/min, and the elution was carried out at a detected wavelength of 210 nm. Using a 10% aqueous acetonitrile solution containing 0.05% trifluoroacetic acid as eluent A, and a 40% aqueous acetonitrile solution containing 0.05% TFA as eluent B, the elution was carried out with a linear gradient from eluent A to eluent B for 24 minutes.

(Measurement of Mass Spectrum)

The mass spectrum was measured with a matrix-assisted laser desorption ionization time-of-flight mass spectrometer (trade name: Voyager-DE STR (manufactured by Applied Biosystems)) using $\alpha$-cyano-4-hydroxycinnamic acid (CHCA) as a matrix.

(Analysis of Ligand Molecule-Introduced ssDNA (Conjugate) According to Reversed Phase HPLC)

A conjugate was analyzed according to reversed phase HPLC under the following conditions. A column under the trade name of OligoDNA RP (manufactured by Tosoh Corporation) was used. The flow rate of an eluent upon elution was 1.0 mL/min, and the elution was carried out at a detected wavelength of 260 nm. A 0.1 M aqueous ammonium acetate solution containing 5% acetonitrile was used as eluent A, and a 0.1 M aqueous ammonium acetate solution containing 30% acetonitrile was used as eluent B. The elution was carried out only with eluent A for 5 minutes and thereafter with a linear gradient from eluent A to eluent B for 50 minutes.

(Measurement of Concentration of ssDNA)

Twenty-five microliters of an ssDNA solution was added to 975 $\mu$L of a 10 mM Tris-HCl buffer solution (pH = 7.0) to dilute. The absorbance at 260 nm was determined with a spectrophotometer provided with a base correction only with the 10 mM Tris-HCl buffer solution. A value obtained by multiplying the absorbance found above by a factor of 40 was defined as an OD value of the ssDNA solution. A weight of ssDNA per OD ($\mu$g) used in Examples can be respectively calculated from a calculated value. The above OD value can be converted to the molar concentration by using the molecular weight of ssDNA.

(Measurement of Concentration of Ligand Construct Having DNA Spacer)

The concentrations of the ligand constructs shown in the following Examples were measured using a DNA Quantification Kit under the trade name of PicoGreen (manufactured by Pierce). The calibration curve was drawn using $\lambda$DNA standard solutions of dsDNA of already known concentrations. The listed found values are an average value of measurements for a total of 3 runs.

(Evaluation of Property of Regulating Function of Cells, Owned by Ligand Construct)

A property of regulating function of the cells was evaluated by comparing function of differentiating immature osteoblasts derived from mouse (MC3T3-E1 cells) into mature osteoblasts. MC3T3-E1 cells were cultured in $\alpha$-MEM (manufactured by GIBCO, $\alpha$-MEM being an abbreviation for Alpha-Minimum Essential Medium) medium to which 10% by weight fetal bovine serum (FBS) had been added. Thereafter, the cultured MC3T3-E1 cells were collected by trypsin treatment in a condition of 90% confluence. The above MC3T3-E1 cells were redispersed in $\alpha$-MEM to which 10 mM $\beta$-glycerophosphoric acid (manufactured by SIGMA), 50 $\mu$g/mL ascorbic acid (manufactured by SIGMA), and 10% by weight FBS (manufactured by SIGMA) had been added, so as to have a density of 19,000 cells/mL. Thereafter, the above MC3T3-E1 cells were sown on a 12-well plate immobilized with a ligand construct in the manner shown hereinbelow so as to be added in a volume of 2 mL per one well. The cell density at this point was 5,000 cells/cm$^2$. The above MC3T3-E1 cells were cultured at 37°C, in a damp air containing 5% by volume $CO_2$. The medium was exchanged every 3 days. At the seventh day from the day of sowing, the alkaline phosphatase (ALP) activity of the MC3T3-E1 cells was determined. A property of regulating function of the cells, owned by the ligand construct, was evaluated using the ALP activity as an index of the extent of differentiation of the MC3T3-E1 cells.

The cells after the culture were collected by trypsin treatment, and redispersed in 0.5 mL of PBS. The cell number was counted with a Burker-Turk counting chamber using the dispersion obtained. Thereafter, as an index of differentiation, the ALP activity of the cells was assayed. The ALP activity was assayed in accordance with p- nitrophenyl phosphate (pNPP) method using a substrate solution [trade name: FAST p-NITROPHENYL PHOSPHATE TABLET SETS (manufactured by SIGMA) was dissolved and used]. Here, utilizing that a substrate pNPP becomes p-nitrophenol by ALP and shows a yellow color, the value of enzyme activity was shown with the amount of p-nitrophenol generated in a certain period of time. A calibration curve was drawn using solutions obtained by dissolving p-nitrophenol of known amounts in PBS, and the amount of p-nitrophenol produced was quantified using the above calibration curve. In addition, a surfactant Triton™ X-100 (manufactured by SIGMA) was used for lysing cell membranes. One-hundred microliters of the cell solution or the calibration curve solution, 50 $\mu$L of 0.3% Triton™ X-100, and 100 $\mu$L of the substrate solution were added to a 96-well plate, and the resulting mixture was incubated at 37°C for 1 hour, to carry out a reaction. Fifty microliters of a 3 N aqueous sodium hydroxide solution was added to the resulting reaction mixture, to stop the enzyme reaction. Thereafter, the absorbance at 450 nm was determined for the resulting product with a microplate reader (GENious manufactured by Tecan Trading AG). The ALP activity per one cell (production rate of p-nitrophenol per one cell (fmol/minute/cell)) was calculated.

One of the simplest embodiments in the present invention is exemplified in the following Examples. In other words, the following Example describes an embodiment where KDGEA peptide (ligand molecule derived from type I collagen) and GRGDS peptide (ligand molecule derived from fibronectin) are used as ligand molecules, and dsDNA having a double helical structure forming a complementary base pair is used as a spacer structure for connecting the ligand molecules, and biotin is used as a functional group for binding substrates.

The positions of introducing two ligand molecules were 5' terminals which were located at two sites in dsDNA. Also, the position of introducing biotin, an active group for binding substrates was between the GRGDS ligand molecule and the dsDNA spacer, the biotin being introduced via a linker. The length of the DNA spacer was 20 nm (60mer each as a double-stranded DNA). A structure of the ligand construct designed according to the above technical idea (schematic view) is shown below. The ligand construct is referred to as KDGEA-SP20-(Bio)GRGDS. A scheme of the synthesis process is as shown in Figure 1.

**[Ka 1]**

[Example 1] Preparation of KDGEA-SP20-(Bio)GRGDS

According to Premodification Method

First, a conjugate in which ligand molecules were bound to ssDNA was synthesized. In the following Example, a maleimide group was selected as a functional group for binding to a nucleic acid spacer contained in the ligand molecule. Specifically, a maleimidized KDGEA having introduction of a maleimide group into an N-terminal of KDGEA, and a maleimidized (Bio)GRGDS having introduction of a maleimide group into an ε-amino group of an amino acid lysine and a biotin group into the N-terminal via a linker, with respect to a peptide molecule KGRGDS having further introduction of the lysine into the N-terminal of GRGDS, were used.
In addition, in the ssDNA, one having introduction of a thiol group into 5' terminal was used as a functional group for binding the ligand molecules. More specifically explaining the structure, it is a synthetic ssDNA in a state of binding an OH site of 6-melcapto-1-hexanol to a phosphate group at a 5' terminal. Immediately after the synthesis, the thiol site at a 5' terminal is protected by a protecting group having a thiol group and a disulfide bond. The above thiol site is deprotected to form a thiol group, and a maleimidized KDGEA or a maleimidized (Bio)GRGDS is introduced thereinto to form a conjugate with ssDNA.

(Synthesis of Maleimidized KDGEA)

A synthetic reaction was started using a resin having previous introduction of Fmoc-Ala (Fmoc being an abbreviation of 9-fluorenylmethoxycarbonyl) (manufactured by Shimadzu Corporation, trade name: Preloaded HMP resin). The deprotection of the Fmoc group was carried out using a solution of 20% piperidine (manufactured by Applied Biosystems)/dimethylformamide (which may be hereinafter abbreviated as DMF). In addition, a condensation reaction for extension of a peptide chain was carried out by dissolving in and adding to DMF 10 equivalents of Fmoc-aa (aa representing an appropriately protected amino acid) and 10 equivalents of HBTU (manufactured by Shimadzu Corporation, HBTU being an abbreviation of 2-(1-H-benzotriazol-1-yl-1,1,3,3-tetramethyluronium hexafluorophosphate) as reagents for condensation, 10 equivalents of HOBt (manufactured by PEPTIDE INSTITUTE, HOBt being an abbreviation of 1-Hydroxybenzotriazole) and 20 equivalents of DIEA (manufactured by Applied Biosystems, DIEA being an abbreviation of Diisopropylethylamine), based on the amount of amino acids in the resin, whereby synthesizing a KDGEA peptide on the resin. The Fmoc group at the N-terminal was deprotected with 20% piperidine/DMF, and 5 equivalents of GMBS (manufactured by DOJINDO LABORATORIES, GMBS being an abbreviation of N-(4-maleimidobutyryloxy)succinimide) and 10 equivalents of DIEA, based on the generated free N-terminal amino group, were dissolved in DMF and added thereto. The mixture was reacted at room temperature for 4 hours, an additional 1 equivalent of GMBS was then added thereto, and the mixture was reacted at room temperature for 1 hour. A resin retaining the resulting product was washed with DMF, and then with methanol and diethyl ether, and the resin was dried under a reduced pressure. A 90% trifluoroacetic acid (manufactured by PEPTIDE INSTITUTE)/5% thioanisole (manufactured by Aldrich)/3% $H_2O$/2% anisole (V/V/V/V) solution was used for cleaving a peptide containing maleimide groups coupled as described above. The above solution was mixed with the resin, and the mixture was stirred at room temperature for 2 hours. Thereafter, the resin was separated by filtration, and the filtrate was concentrated under a reduced pressure. A cooled diethyl ether was added to the residue to precipitate a peptide, and a solid was separated by filtration and dried under a reduced pressure. The resulting peptide was purified by HPLC for purification under the conditions mentioned above. It was confirmed that the isolated peptide appeared in a single peak (retention time: 10.4 minutes) by HPLC for analysis (condition a). In addition, the molecular weight of the above peptide was measured by mass spectrum. As a result, m/z = 684.60 [M+H]$^+$, which was consistent with the calculated value. The resulting product was lyophilized to give a maleimidized KDGEA in the form of solid.

(Synthesis of Maleimidized (Bio)GRGDS)

GRGDS peptide was synthesized on the resin in the same manner as in the maleimidized KDGEA using a resin having previous introduction of Fmoc-Ser (manufactured by Shimadzu Corporation, trade name: Preloaded HMP resin). The Fmoc group at an N-terminal of GRGDS peptide was deprotected, and a protected amino acid Fmoc-Lys (ivDde)-OH (manufactured by Novabiochem, Fmoc-Lys(ivDde)-OH representing N-$\alpha$-Fmoc-N-$\epsilon$-1-(4,4-dimethyl-2,6-dioxocyclohex-1-ylidene)-3-methylbutyl-L-lysine) was introduced thereinto, according to a condensation reaction for extension of a peptide chain. Further, the Fmoc group at the N-terminal was deprotected, and a protected amino acid Fmoc-AEEA (manufactured by PEPTIDES INSTITUTE, Fmoc-AEEA representing Fmoc-8-amino-3,6-dioxaoctanoic acid) was repeatedly introduced thereinto twice as a linker site, according to a condensation reaction for extension of a peptide chain. The Fmoc group at the N-terminal was deprotected, and 5 equivalents of Biotin-OSu (manufactured by Novabiochem, Biotin-OSu representing Biotin N-hydroxysuccinimide ester) and 10 equivalents of DIEA, based on the generated free N-terminal amino group, were dissolved in DMF and added thereto. The mixture was reacted at room temperature for 4 hours, an additional 1 equivalent of Biotin-OSu was then added thereto, and the mixture was reacted at room temperature for 1 hour. The resulting product was washed with DMF, and thereafter deprotected with a 2% hydrazine/DMF solution, and an $\epsilon$-amino group was generated in the lysine residue. Five equivalents of GMBS (manufactured by DOJINDO LABORATORIES, GMBS being an abbreviation of N-(4-maleimidobutyryloxy)succinimide) and 10 equivalents of DIEA, based on the generated free $\epsilon$-amino group, were dissolved in DMF and added thereto. The mixture was reacted at room temperature for 4 hours, an additional 1 equivalent of GMBS was then added thereto, and the mixture was reacted at room temperature for 1 hour. A resin retaining the resulting product was washed with DMF, and then with methanol and diethyl ether, and the resin was dried under a reduced pressure. A 90% trifluoroacetic acid (manufactured by PEPTIDE INSTITUTE)/5% thioanisole (manufactured by Aldrich)/3% H$_2$O/2% anisole (V/V/V/V) solution was used for cleaving a peptide containing maleimide groups coupled as described above. The above solution was mixed with the resin, and the mixture was stirred at room temperature for 2 hours. Thereafter, the resin was separated by filtration and the filtrate was concentrated under a reduced pressure. A cooled diethyl ether was added to the residue to precipitate a peptide, and a solid was separated by filtration and dried under a reduced pressure. The resulting crude peptide was purified by HPLC for purification under the conditions mentioned above. It was confirmed that the isolated peptide appeared in a single peak (retention time: 14.8 minutes) by HPLC for analysis (condition b). In addition, the molecular weight of the above peptide was measured by mass spectrum. As a result, m/z = 1301.56 [M+H]$^+$, which was consistent with the calculated value. The resulting product was lyophilized to give a maleimidized (Bio)GRGDS in the form of solid.

(Deprotection of ssDNA Containing Thiol Group)

Thiol group-protected ssDNA, that serves as a raw material for KDGEA-SP20-(Bio)GRGDS, are two kinds, PS-S-SP20A and PS-S-SP20D (see Figure 1). PS-S-SP20A (manufactured by Sigma Genosys) was dissolved in TE buffer (10 mM Tris-HCl buffer solution containing 1 mM ethylenediaminetetraacetic acid (which may be hereinafter described as EDTA), pH = 8.0), so as to have a concentration of 100 nmol/mL. The amount 0.2 mL of a 0.08 M dithiothreitol solution (prepared by dissolving dithiothreitol in a 0.25 M phosphate buffer solution (pH 8.0)) was mixed with 0.2 mL of the resulting solution, and the mixture was stirred at room temperature for 16 hours. The resulting mixture was purified by a column under the trade name of NAP-5 column (manufactured by Pharmacia Biotech) equilibrated with an eluent (0.1 M phosphate buffer (pH 6.0)), to give HS-SP20A having deprotection of a thiol group. The concentration of HS-SP20A was determined by measuring absorbance at 260 nm (see Table 1). PS-S-SP20D was also deprotected in the same manner as the above, to give HS-SP20D (see Table 1).
[Table 1]

| Deprotected ssDNA | $\mu$g/OD | nmol/OD | Concentration (nmol/mL) |
|---|---|---|---|
| HS-SP20A | 31.5 | 3.12 | 19.1 |
| HS-SP20D | 31.6 | 3.13 | 13.9 |

(Preparation of KDGEA-S-SP20A and (Bio)GRGDS-S-SP20D)

A method of preparing KDGEA-S-SP20A according to the binding formation of the maleimidized KDGEA to HS-SP20A obtained by deprotection as described above (see Figure 1) will be described. The amount 0.8 mL of a HS-SP20A solution (concentration being 12.5 nmol/mL) was mixed with a solution prepared by dissolving in 0.8 mL of 0.1 M phosphate buffer (pH 7.0) the maleimidized KDGEA in an amount of 25 equivalents based on the HS-SP20A, and the mixture was reacted at 4°C for 24 hours. This reaction solution was lyophilized, and thereafter a lyophilized product was dissolved in 0.5 mL of a 10 mM Tris-HCl buffer solution containing 1 mM EDTA (pH = 7.0). KDGEA-S-SP20A was purified by gel filtration with NAP-5 (trade name) equilibrated with an eluent. Here, as the above eluent, a 10 mM Tris-HCl buffer solution containing 1 mM EDTA (pH = 7.0) was used. The absorbance at 260 nm was measured and the concentration was calculated utilizing the data of HS-SP20A (Table 1) (see Table 2). (Bio)GRGDS-S-SP20D, a conjugate of the maleimidized (Bio)GRGDS and HS-SP20D, was obtained in the same manner as the above (see Table 2). It was confirmed by HPLC that all the conjugates appeared in a single peak.

[Table 2]

| Conjugate | Concentration (nmol/mL) |
|---|---|
| KDGEA-S-SP20A | 21.0 |
| (Bio)GRGDS-S-SP20D | 9.8 |

(Preparation of KDGEA-SP20-(Bio)GRGDS, Ligand Construct Containing Biotin Group)

KDGEA-S-SP20A, (Bio)GRGDS-S-SP20D, SP20C, and a buffer solution were mixed as in Table 3, and the mixture was incubated at 94°C for 30 seconds, and then at 55°C for 30 seconds (see Figure 1), thereby annealing the mixture. Ninety microliters of the solution was taken out therefrom and mixed with 90 $\mu$L of an enzyme solution of a DNA ligation kit (trade name: DNA Ligation Kit (Ver. 1), manufactured by TAKARA BIO, INC.), and the ligation reaction was carried out at 16°C for 60 minutes. The reaction mixture was purified by a spin-column (trade name: QIA PCR Purification Kit, manufactured by QIAGEN), to give KDGEA-SP20-(Bio)GRGDS. The mixture was detected by polyacrylamide gel electrophoresis in accordance with the conventional method, and as a result, it could be confirmed that a nucleic acid spacer of the resulting KDGEA-SP20-(Bio)GRGDS was a 60mer as dsDNA. It was seen from the above that a spacer site comprising dsDNA could be synthesized with a length as designed. The concentration was quantified with one under the trade name of PicoGreen. As a result, the concentration was 21.0 $\mu$g/mL. Assuming that the molecular weight of dsDNA portion of the KDGEA-SP20-(Bio)GRGDS is about 36,800, the concentration is calculated to be 0.57 nmol/mL.

[Table 3]

| Reagents | Concentration (nmol/mL) | Amount ($\mu$L) |
|---|---|---|
| KDGEA-S-SP20A | 21.0 | 23.8 |
| (Bio)GRGDS-S-SP20D | 9.8 | 51.2 |
| SP20C | 100 | 10.0 |
| Buffer Solution | *1 | 15.0 |
| Total | - | 100.0 |

*1; 1 M Tris-HCl (pH 7.6), 50 mM MgCl$_2$, 1 M NaCl

[Production Example 1]

(Preparation of Surface 1 Immobilized with Streptavidin)

Streptavidin (which may be hereinafter abbreviated as SA) manufactured by SIGMA was dissolved in a 0.1 M aqueous sodium hydrogencarbonate solution, so as to have a concentration of 100 $\mu$g/mL. This solution was added to a 12-well plate made of non-treated polystyrene (manufactured by Becton, Dickinson and Company) in a volume of 0.5 mL/well, and SA was adsorbed to the surface at 27°C for 1 hour. Each well was washed 3 times with 1 mL of PBS, and thereafter the immobilization density of streptavidin was measured with a protein assay kit manufactured by Pierce (trade name: BCA Protein Assay Kit). As a result, the immobilization density of streptavidin was 0.32 $\mu$g/cm$^2$ (5.3 pmol/cm$^2$).

Incidentally, the amount of SA immobilized and the immobilization density thereof were calculated from protein concentrations of the SA solutions before and after binding determined with the above protein assay kit. In other words, the amount of SA reduced per well ($\mu$g) corresponds to the amount of SA immobilized, and the immobilization density is calculated by dividing the amount immobilized by the coating area 4.5 cm$^2$ (the reason therefor being in a case a 12-well plate is coated with 0.5 mL of the SA solution, the coating area is 4.5 cm$^2$). In addition, the molar density per unit area was calculated, assuming that the molecular weight of SA is 60,000. Also, as a blocking agent, a PBS solution containing 1% bovine serum albumin (Fraction V manufactured by SIGMA was used; hereinafter abbreviated as BSA) was added thereto in a volume of 1 mL/well, and the blocking agent was adsorbed to the surface at 27°C for 1 hour. The resulting surface was washed 3 times with 1 mL of PBS, to give a surface 1 immobilized with SA.

[Production Example 2]

(Preparation of Surface 2 Immobilized with SA on BSA Coating Layer)

A PBS solution containing 1% BSA was added to a delta-treated 12-well plate (manufactured by NUNC) in a volume of 0.5 mL per one well, and BSA was adsorbed at room temperature for 1 hour. The resulting mixture was washed 3 times with 1 mL of PBS, and thereafter a PBS solution containing 20 $\mu$M EZ-Link NHS-PEO$_4$-Biotin (manufactured by Pierce) was added thereto in a volume of 0.5 mL per well, and the mixture was reacted at room temperature for 2 hours. The reaction mixture was washed 3 times with 1 mL of PBS, and thereafter a PBS solution containing 15 $\mu$g/mL SA was added thereto in a volume of 0.5 mL, and SA was adsorbed at room temperature for 5 hours. The immobilization density of SA was measured in the same manner as in Production Example 1. As a result, the immobilization density of streptavidin was 0.48 $\mu$g/cm$^2$ (8 pmol/cm$^2$). The resulting surface was washed 3 times with 1 mL of PBS, to prepare a surface 2 immobilized with SA on a BSA coating layer.

[Production Example 3]

(Preparation of Surface 3 Immobilized with SA on BSA Coating Layer)

Three milligrams of BSA was dissolved in 2.85 mL of PBS, and thereafter 0.15 mL of a solution prepared by dissolving 20 $\mu$mol Sulfo-NHS-LC-biotin (manufactured by Pierce) in DMF was added thereto, and the mixture was shaken at room temperature for 1 hour to be reacted. The solution after the reaction was transferred to a dialyzer, and dialyzed 3 times against a PBS solution containing 300 mL of 0.05% NaN$_3$ at 4°C, to give a biotinylated BSA. A 20 $\mu$g/mL biotinylated BSA solution was added to a delta-treated 12-well plate (manufactured by NUNC) in a volume of 0.5 mL per well, and allowed to stand overnight at room temperature, to adsorb the biotinylated BSA to the plate. The resulting mixture was washed 3 times with PBS, and thereafter a PBS solution containing 1% BSA was added to each well in a volume of 0.5 mL, and the mixture was allowed to stand at room temperature for 1 hour, and then again washed 3 times with PBS. Next, a 16.8 $\mu$g/mL PBS solution containing SA was added in a volume of 0.5 mL per one well, and the mixture was shaken at room temperature for 5 hours to immobilize SA. Thereafter, the resulting mixture was washed 3 times with PBS, to prepare a substrate moiety immobilized with SA on a BSA coating layer. The immobilization density of SA was measured in the same manner as in Production Example 1. As a result, the immobilization density of SA was 0.54 $\mu$g/cm$^2$ (9 pmol/cm$^2$). The resulting surface was washed 3 times with 1 mL of PBS, to prepare a surface 3 immobilized with SA on a BSA coating layer.

[Production Example 4]

(Preparation of Surface 4 Immobilized with SA on Alginate Hydrogel Coating Layer)

A 0.1% aqueous sodium alginate (manufacture by Kibun Food Chemifa Co., Ltd., trade name: NSPH) solution was added to a glass dish (having a diameter of 2.7 cm) in a volume of 0.5 mL per one well, and dried at room temperature. A 1% calcium chloride solution was added to the above well in a volume of 0.5 mL per one well, and alginic acid was cured at room temperature for 2 hours. Thereafter, PBS was added thereto in a volume of 1 mL per one well, the mixture was allowed to stand for 3 minutes, and a washing procedure with aspiration was carried out 3 times, to form an alginate hydrogel coating layer. Four-hundred and twelve milligrams of EDC·HCL (manufactured by PEPTIDES INSTITUTE) was dissolved in 20 mL of DMF containing 250 mg of N-hydroxysuccinimide. This solution was added thereto in a volume of 1 mL per one well, and the mixture was reacted at room temperature for 20 hours. The reaction mixture was washed 3 times with 1 mL of methanol per a dish, and dried. DMF containing 100 mM ethylene diamine was added thereto in a volume of 1 mL per one well, and the mixture was reacted at room temperature for 6 hours. The reaction mixture was washed 3 times with 1 mL of PBS, and thereafter a PBS solution containing 20 $\mu$M EZ-Link NHS-PEO$_4$-Biotin (manu-

factured by Pierce) was added thereto in a volume of 0.5 mL, and the mixture was reacted at room temperature for 2 hours. The reaction mixture was washed 3 times with 1 mL of PBS, a PBS solution containing 15 μg/mL SA was then added thereto in a volume of 0.5 mL, and the mixture was immobilized at room temperature for 5 hours. The resulting mixture was allowed to stand, and thereafter washed again 3 times with 1 mL of PBS. The immobilization density of SA was measured in the same manner as in Production Example 1. As a result, the density of bound SA was 0.28 μg/cm$^2$ (4 pmol/cm$^2$). The resulting surface was washed 3 times with 1 mL of PBS, to prepare a substrate moiety having a surface 4 immobilized with SA on an alginate hydrogel coating layer.

[Examples 2 to 5] Preparation of Base Material for Cell Culture

KDGEA-SP20-(Bio)GRGDS prepared in Example 1 was dissolved in PBS containing 1 mM EDTA, so as to have a concentration shown in Table 4, with regard to each surface. This solution was sterilized by filtrating with a filter of 0.2 μm, and added to the surfaces 1 to 4 prepared in Production Examples 1 to 4 in a volume of 0.5 mL per one well. Incidentally, the immobilization density of the ligand construct is not limited to those of the present Examples, and the immobilization density can be adjusted by increasing and decreasing the concentration of the fed KDGEA-SP20-(Bio) GRGDS. Thereafter, the ligand constructs were immobilized at 4°C over 12 hours. The concentrations of dsDNA of ligand construct solutions before and after the immobilization reaction were measured with one under the trade name of PicoGreen. From the difference of both the concentrations, the extent of which the ligand constructs added was immobilized to each surface was calculated. The results are shown in Table 4.

[Table 4]

| Surface | Concentration Before Immobilization[*1] (ng/ 0.5 mL) | Concentration After Immobilization (ng/ 0.5 mL) | Immobilization Ratio (%)[*2] | Density of KDGEA-SP20-(Bio)GRGDS at Surface (fmol/cm$^2$)[*3] |
|---|---|---|---|---|
| 1 (Ex. 2) | 53 | 45 | 15 | 47 |
| 2 (Ex. 3) | 19 | 3.8 | 80 | 89 |
| 3 (Ex. 4) | 19 | 3.6 | 81 | 90 |
| 4 (Ex. 5) | 20 | 6.0 | 70 | 82 |

*1; A value quantified according to PicoGreen, approximately 0.45 pmol/0.5 mL if calculated in terms of a molar concentration.

2; The immobilization ratio is calculated by: *3; The density is calculated by:

$$\text{Immobilization Ratio (\%)} = \left(1 - \frac{\text{Concentration After Immobilization}}{\text{Concentration Before Immobilization}}\right) \times 100$$

$$\frac{\dfrac{\text{Weight of Ligand Construct Immobilized (ng)}}{\text{Molecular Weight of Ligand Construct (36,800)}}}{\text{Area of Well (4.5 cm}^2\text{)}} \times 10^6$$

As shown in Table 4, a ligand construct containing a functional group for binding substrates was efficiently immobilized to a base material having a functional group for immobilizing the ligand construct. Especially, as a combination of a functional group for binding substrates to a base material and a functional group for immobilizing a ligand construct, in a case of having a combination of streptavidin-biotin, a ligand construct was immobilized in a high efficiency, regardless of the kinds of the materials for the substrates and the coating layers.

[Test Example 1]

(Evaluation of Property of Regulating Cell Functions Owned by Ligand Construct)

Using the surfaces 1 to 4 of the substrates for cell culture prepared in Examples 2 to 5, and the surfaces 1 to 4 without immobilization of the ligand construct, the property of regulating cell functions was evaluated. The evaluation was carried out using as an index an ability of differentiating immature osteoblasts derived from mouse (MC3T3-E1 cell) into mature osteoblasts (ALP activity) by the ligand construct as described in the above (Evaluation of Property of Regulating Cell Functions Owned by Ligand Construct). The results of ALP activity per one cell measured on the seventh day of the culture of MC3T3-E1 cells are shown in Table 5.

[Table 5]

| Kinds of Surface on Substrate for Cell Culture | ALP Activity (fmol/min/cell)[*1] |
|---|---|
| Surface 1 | 51 (28) |
| Surface 2 | 35 (25) |
| Surface 3 | 38 (23) |
| Surface 4 | 37 (-[*2]) |

*1 The numerical figures in parentheses is the determination result of ALP activity at surface without immobilization of KDGEA-SP20-(Bio)GRGDS.
*2 "-" means that the cells are removed from the surface before the seventh day, so that ALP activity could not be evaluated.

As shown in Table 5, MC3T3-E1 cells cultured at surfaces having KDGEA-SP20-(Bio)GRGDS showed a markedly amplified ALP activity, as compared to that of cells cultured at surfaces without having KDGEA-SP20-(Bio)GRGDS.

[Comparative Production Example 1]

(Synthesis of Maleimidized GRGDS)

A maleimidized GRGDS peptide was synthesized in the same manner as in the maleimidized KDGEA of Example 1, using a resin having previous introduction of Fmoc-Ser (manufactured by Shimadzu Corporation, trade name: Preloaded HMP resin). The resulting crude peptide was purified by HPLC for purification under the conditions mentioned above. It was confirmed that an isolated peptide appeared in a single peak (retention time: 8.6 minutes) by HPLC for analysis (condition **a**). In addition, the molecular weight of the above peptide was measured according to mass spectrum. As a result, m/z = 656.61 [M+H]$^+$, which was consistent with the calculated value. The resulting product was lyophilized to give a solid maleimidized GRGDS.

(Deprotection of ssDNA Containing Thiol Group)

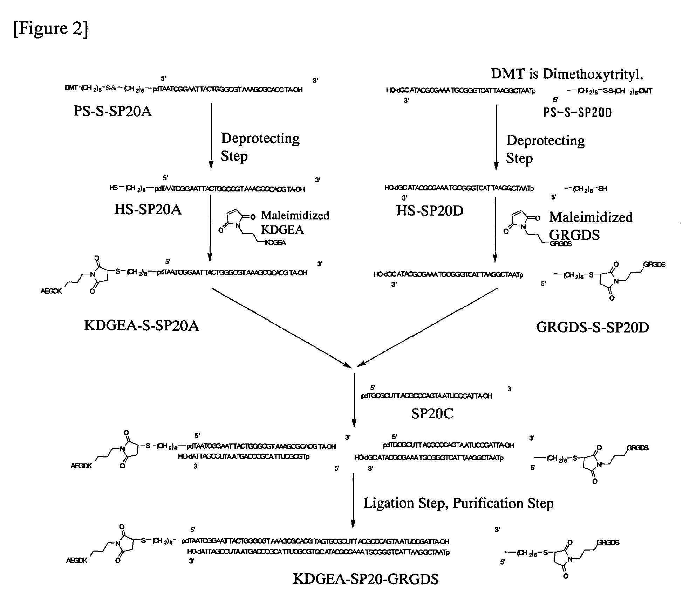

A thiol group-protected ssDNA's, that serve as raw materials for KDGEA-SP20-GRGDS are two kinds, PS-S-SP20A and PS-S-SP20D (see Figure 2). PS-S-SP20A (manufactured by Sigma Genosys) was dissolved in TE buffer (a 10 mM Tris-HCl buffer solution containing 1 mM EDTA, pH = 8.0), so as to have a concentration of 100 nmol/mL. The amount 0.2 mL of a 0.08 M dithiothreitol solution (prepared by dissolving dithiothreitol in a 0.25 M phosphate buffer solution (pH 8.0)) was mixed with 0.2 mL of the resulting solution, and the mixture was stirred at room temperature for 16 hours. The resulting mixture was purified by a column under the trade name of NAP-5 column manufactured by Pharmacia Biotech, equilibrated with an eluent, to give HS-SP20A of which thiol group was deprotected. Here, a 0.1 M phosphate buffer (pH 6.0) was used as the eluent. The concentration of HS-SP20A was determined by measuring the absorbance at 260 nm (see Table 6). PS-S-SP20D was also deprotected in the same manner as the above, to give HS-SP20D (see Table 6).

[Table 6]

| Deprotected ssDNA | μg/OD | nmol/OD | Concentration (nmol/mL) |
|---|---|---|---|
| HS-SP20A | 31.5 | 3.12 | 19.1 |
| HS-SP20D | 31.6 | 3.13 | 13.7 |

(Preparation of KDGEA-S-SP20A and GRGDS-S-SP20D)

A method of preparing KDGEA-S-SP20A according to the binding formation of the maleimidized KDGEA to the HS-SP20A obtained by the deprotection as mentioned above (see Figure 2) will be described. The amount 0.8 mL of a 17.0 nmol/mL HS-SP20A solution and 25 equivalents of the maleimidized KDGEA (prepared in Example 1) based on the HS-SP20A were dissolved in 0.8 mL of a 0.1 M phosphate buffer (pH 7.0). The resulting solution was mixed and reacted at 4°C for 24 hours. This reaction solution was lyophilized, and a lyophilized product was then dissolved in 0.5 mL of a 10 mM Tris-HCl buffer solution containing 1 mM EDTA (pH = 7.0). KDGEA-S-SP20A was purified by gel filtration with one under the trade name of NAP-5 equilibrated with an eluent. Here, as the eluent, a 10 mM Tris-HCl buffer solution containing 1 mM EDTA (pH = 7.0) was used. The absorbance at 260 nm was measured, and the concentration of KDGEA-S-SP20A was calculated utilizing the data for HS-SP20A (Table 6) (see Table 7). GRGDS-S-SP20D, a conjugate of the maleimidized GRGDS and the HS-SP20D, was obtained in the same manner as the above (see Table 7). It was confirmed by HPLC that all the conjugates appeared in a single peak.

[Table 7]

| Conjugate | Concentration (nmol/mL) |
|---|---|
| KDGEA-S-SP20A | 21.0 |
| GRGDS-S-SP20D | 18.0 |

(Preparation of KDGEA-SP20-GRGDS)

KDGEA-S-SP20A, GRGDS-S-SP20D, SP20C, a buffer solution, and water were mixed in a ratio as shown in Table 8. The resulting solution was incubated at 94°C for 30 seconds, and then at 55°C for 30 seconds, thereby annealing the solution. Ninety microliters of the solution was taken out therefrom and mixed with 90 $\mu$L of an enzyme solution of a DNA ligation kit (trade name: DNA Ligation Kit (Ver. 1), manufactured by TAKARA BIO, INC.), and the ligation reaction was carried out at 16°C for 60 minutes. The resulting product was purified by a spin-column (trade name: QIA PCR Purification Kit, manufactured by QIAGEN), to give KDGEA-SP20-GRGDS. When the product was detected by polyacrylamide gel electrophoresis in accordance with the conventional method, it could be confirmed that dsDNA of the resulting KDGEA-SP20-GRGDS is a 60mer. It could be seen from the above that a spacer site comprising dsDNA could be synthesized with a length as designed. The concentration was quantified with one under the trade name of PicoGreen. As a result, the concentration was 48.0 $\mu$g/mL. Assuming that the molecular weight of dsDNA portion of the KDGEA-SP20-GRGDS is about 36,800, the concentration is calculated to be 1.3 nmol/mL.

[Table 8]

| Reagent | Concentration (nmol/mL) | Amount ($\mu$L) |
|---|---|---|
| KDGEA-S-SP20A | 21.0 | 23.8 |
| GRGDS-S-SP20D | 18.0 | 27.8 |
| SP20C | 100 | 10.0 |
| Buffer Solution*1 | -*1 | 10.0 |
| H$_2$O | - | 28.4 |
| Total | - | 100 |

*1; 1 M Tris-HCl (pH 7.6), 50 mM MgCl$_2$, 1 M NaCl

[Comparative Example 1]

(Physical Adsorption of KDGEA-SP20-GRGDS to Polystyrene Dish)

KDGEA-SP20-GRGDS prepared in Comparative Production Example 1 was dissolved in a 10 mM Tris-HCl buffer solution containing 2 mM EDTA, (pH = 7.4), so as to have a concentration of 0.9 pmol/mL. The resulting solution was added to a surface 1 prepared in Production Example 1 in a volume of 0.5 mL per 1 well. In the case of a 12-well plate, when a liquid is added to 1 well in a volume of 0.5 mL, an area covered by the liquid is about 4.5 cm$^2$. Therefore, the density if all the ligand constructs bind to the surface 1 in this case is 100 fmol/cm$^2$. Thereafter, the ligand constructs were adsorbed at 4°C over 12 hours. The concentrations of dsDNA of the ligand construct solutions before and after the adsorption were measured with one under the trade name of PicoGreen. From the difference of both the concentrations, the extent of which the ligand constructs added were immobilized to a surface 1 was calculated. As a result, it could be seen that the KDGEA-SP20-GRGDS cannot be immobilized.

[Comparative Example 2]

(Physical Adsorption of KDGEA-SP20-GRGDS to Alginate Hydrogel Coating Layer)

KDGEA-SP20-GRGDS prepared in Comparative Production Example 1 was dissolved in a 10 mM Tris-HCl buffer solution containing 2 mM EDTA, pH = 7.4), so as to have a concentration of 0.9 pmol/mL. The resulting solution was added to a surface 4 prepared in Production Example 4 in a volume of 0.5 mL per 1 well. Thereafter, the ligand constructs were adsorbed at 4°C over 12 hours. The concentrations of dsDNA of the ligand construct solutions before and after the adsorption were measured with one under the trade name of PicoGreen. From the difference of both the concentrations, the extent of which the ligand constructs added were immobilized to a surface 4 was calculated. As a result, it could be seen that the KDGEA-SP20-GRGDS cannot be immobilized.

The following example describes an embodiment where KDGEA peptide (ligand molecule derived from type I collagen) and GRGDS peptide (ligand molecule derived from fibronectin) are used as ligand molecules, dsDNA having a double helical structure forming a complementary base pair is used as a spacer structure coupling with the ligand molecules, and a thiol group is used as a functional group for binding substrates.

The positions at which the two ligand molecules were introduced were 5' terminals located at two sites in dsDNA. Also, the position into which a thiol group that was an active group for binding substrates was introduced was between the GRGDS ligand molecule and the dsDNA spacer. The length of the DNA spacer was 20 nm (60mer each as a double-stranded DNA). A ligand construct designed according to the above technical ideas (schematic view) is shown below. The ligand construct is referred to as KDGEA-SP20-(C)GRGDS.

[Example 6] Preparation of KDGEA-SP20-(C)GRGDS According to Premodification Method

First, a conjugate in which ligand molecules were bound to ssDNA was synthesized. The maleimidized KDGEA (Example 1) was used as one of ligand molecules containing a functional group for binding to a nucleic acid spacer in the same manner as in Example 1. As the other ligand molecule containing a functional group for binding to a nucleic acid spacer, a maleimidized (C)GRGDS obtained by introducing a maleimide group into an ε-amino group of the cysteine with respect to a peptide molecule (C)GRGDS having a further introduction of cysteine into an N-terminal of GRGDS. A conjugate with ssDNA was formed in the same manner as in Example 1 using these ligand molecules. Here, a thiol group of the cysteine is used as an active group for binding substrates.

(Synthesis of Maleimidized (CP)GRGDS)

GRGDS peptide was synthesized on the resin in the same manner as in the maleimidized KDGEA of Example 1, using a resin having previous introduction of Fmoc-Ser (manufactured by Shimadzu Corporation, trade name: Preloaded HMP resin). The Fmoc group at an N-terminal of GRGDS peptide was deprotected, and a protected amino acid Fmoc-Cys(tButhio)-OH (manufactured by Novabiochem, Fmoc-Cys(tButhio)-OH representing N-α-Fmoc-S-t-Butylthio-L-Cysteine) was introduced thereinto according to a condensation reaction for extension of a peptide chain. Further, the Fmoc group at an N-terminal was deprotected, and 5 equivalents of GMBS and 10 equivalents of DIEA, based on the generated free ε-amino group, were dissolved in DMF and added thereto. The mixture was reacted at room temperature for 4 hours, an additional 1 equivalent of GMBS was added thereto, and the mixture was reacted at room temperature for 1 hour. A resin retaining the resulting product was washed with DMF, and thereafter washed with methanol and diethyl ether, and the resin was dried under a reduced pressure. In the cleavage of a peptide containing maleimide groups coupled as described above, a 90% trifluoroacetic acid (manufactured by PEPTIDE INSTITUTE)/5% thioanisole (manufactured by Aldrich)/3% H₂O/2% anisole (V/V/V/V) solution was used. The above solution was mixed with the resin, and the mixture was stirred at room temperature for 2 hours. Thereafter, the resin was separated by filtration, and the filtrate was concentrated under a reduced pressure. A cooled diethyl ether was added to the residue to precipitate the peptide, and a solid was separated by filtration and dried under a reduced pressure. The resulting crude peptide was purified by HPLC for purification under the conditions mentioned above. It was confirmed that an isolated peptide appeared in a single peak (retention time of 19.2 minutes) by HPLC for analysis (condition a). In addition, the molecular weight of the above peptide was measured according to a mass spectrum. As a result, $m/z = 847.66$ $[M+H]^+$, which was consistent with the calculated value. The resulting product was lyophilized to give a maleimidized (CP)GRGDS in the form of solid. Here, the thiol group, an active group for binding substrates, is still protected at this point.

(Preparation of (C)GRGDS-S-SP20D)

(CP)GRGDS-S-SP20D, a conjugate of the maleimidized (CP)GRGDS and the HS-SP20D, was obtained in the same manner as in Example 1. It was confirmed that the conjugate appeared in a single peak by HPLC. The concentration was measured from the absorbance at 260 nm. As a result, the concentration was 10.4 nmol/mL.

(Preparation of Cysteine Group-Containing Ligand Construct KDGEA-SP20-(C)GRGDS)

KDGEA-S-SP20A (Example 1), (CP)GRGDS-S-SP20D, SP20C, and a buffer solution were mixed in a ratio as shown in Table 9, and the mixture was incubated at 94°C for 30 seconds, and then at 55°C for 30 seconds, thereby annealing the mixture. Ninety microliters of the solution was taken out therefrom and mixed with 90 μL of an enzyme solution of a DNA ligation kit (trade name: DNA Ligation Kit (Ver. 1), manufactured by TAKARA BIO, INC.), and the ligation reaction was carried out at 16°C for 60 minutes. The reaction mixture was purified by a spin-column (trade name: QIA PCR Purification Kit, manufactured by QIAGEN), to give KDGEA-SP20-(CP)GRGDS. When the product was detected by polyacrylamide gel electrophoresis in accordance with the conventional method, it could be confirmed that a nucleic acid spacer of the resulting KDGEA-SP20-(CP)GRGDS is a 60mer as dsDNA. It could be seen from the above that a spacer site comprising dsDNA could be synthesized with a length as designed. In addition, in order to deprotect the thiol group of the ligand molecule portion, 0.1 mL of a 0.08 M dithiothreitol solution (prepared by dissolving dithiothreitol in a 0.25 M phosphate buffer solution (pH 8.0)) was mixed with 0.1 mL of the KDGEA-SP20-(CP)GRGDS solution, and the mixture was stirred at room temperature for 16 hours. The resulting mixture was purified by NAP-5 column manufactured by Pharmacia Biotech, equilibrated with an eluent, to give a thiol group-deprotected KDGEA-SP20-(C)GRGDS (0.1 M phosphate buffer (pH 6.0) was used as the eluent). The concentration was quantified with one under the trade name of PicoGreen. As a result, the concentration was 21.7 μg/mL. Assuming that the molecular weight of dsDNA portion of KDGEA-SP20-(C)GRGDS is about 36,800, the concentration is calculated to be 0.59 nmol/mL.
[Table 9]

| Reagents | Concentration (nmol/mL) | Amount (μL) |
|---|---|---|
| KDGEA-S-SP20A | 21.0 | 23.8 |
| (CP)GRGDS-S-SP20D | 10.4 | 51.0 |
| SP20C | 100 | 10.0 |
| Buffer Solution | *1 | 15.2 |
| Total | - | 100.0 |

*1; 1 M Tris-HCl (pH 7.6), 50 mM MgCl₂, 1 M NaCl

[Production Example 5]

(Preparation of Surface 5 Immobilized with Maleimide Group on BSA Coating Layer)

A PBS solution containing 1% BSA was added to a delta-treated 12-well plate (manufactured by NUNC) in a volume of 0.5 mL per 1 well, and BSA was adsorbed at room temperature for 1 hour. The resulting mixture was washed 3 times with 1 mL of PBS, a PBS solution containing 1 mM Sulfo-KMUS (manufactured by Pierce, Sulfo-KMUS representing N-(κ-maleimidoundecanoyloxy)-sulfosuccinimide ester) was then added thereto in a volume of 0.5 mL per well, and the mixture was reacted at room temperature for 1 hour. The reaction mixture was washed 3 times with 1 mL of PBS, to prepare a surface 5 immobilized with a maleimide group on a BSA coating layer.

The immobilization density of the maleimide group was measured as follow. PBS (containing 1 mM EDTA) containing 3 $\mu$M thioethanol was added in a volume of 0.5 mL per 1 well, and the mixture was reacted at room temperature for 14 hours. Thereafter, 0.3 mL of the solution in the well was collected, and mixed with 1.7 mL of a 0.1 mM borate buffer (pH 8.0) containing 0.6 mM ABD-F (manufactured by DOJINDO LABORATORIES, ABD-F representing 4-fluoro-7-sulfamoylbenzofurazan), and the mixture was reacted at 50°C for 5 minutes. The reaction mixture was ice-cooled, and 0.6 mL of a 0.1 N aqueous HCl solution was added thereto, and a fluorescence intensity (excitation wavelength of 380 nm/fluorescence wavelength of 510 nm) was measured. The amount of immobilized thioethanol was calculated from concentrations of thioethanol before and after the reaction. In other words, the amount of thioethanol reduced per well (pmol) corresponds to the amount of immobilized thioethanol, and the immobilization density is calculated by dividing the amount immobilized by a coating area (in the case of a 12-well plate) 4.5 cm$^2$. As a result of the determination, the binding density of thioethanol is 119 pmol/cm$^2$, and this binding density of thioethanol shows the immobilization density of the maleimide group.

[Example 7] Preparation of Base Material for Cell Culture

The thiol group-deprotected KDGEA-SP20-(C)GRGDS prepared in Example 6 was dissolved in PBS containing 1 mM EDTA, so as to have a concentration shown in Table 10, based on a surface 5. This solution was sterilized by filtration with a filter of 0.2 $\mu$m, and added to a surface 5 prepared in Production Example 5 in a volume of 0.5 mL per 1 well. Incidentally, the immobilization density of the ligand construct is not limited to that of the present example, and the density can be adjusted by increasing or decreasing the concentration of the thiol group-deprotected KDGEA-SP20-(C)GRGDS fed. Thereafter, the ligand constructs were immobilized at room temperature for 70 hours or more. The concentrations of dsDNA of ligand construct solutions before and after the immobilization reaction were measured with one under the trade name of PicoGreen. From the difference of both the concentrations, the extent of which the ligand constructs added were immobilized to each surface was calculated.
The results are shown in Table 10.

[Table 10]

| Surface | Concentration Before Immobilization[*1] (ng/0.5 mL) | Concentration After Immobilization (ng/0.5 mL) | Immobilization Ratio (%)[*2] | Density of KDGEA-SP20-(C)GRGDS at Surface (fmol/cm$^2$)[*3] |
|---|---|---|---|---|
| 5 (Ex. 7) | 150 | 142 | 5 | 48 |

*1; A value quantified according to PicoGreen, approximately 0.45 pmol/0.5 mL if calculated in terms of a molar concentration.

*2; The immobilization ratio is calculated by: *3; The density is calculated by:

$$\text{Immobilization Ratio (\%)} = 1 - \frac{\text{Concentration After Immobilization}}{\text{Concentration Before Immobilization}} \times 100$$

$$\frac{\dfrac{\text{Weight of Ligand Construct Immobilized (ng)}}{\text{Molecular Weight of Ligand Construct (36,800)}}}{\text{Area of Well (4.5 cm}^2\text{)}} \times 10^6$$

[Test Example 2]

(Evaluation of Property of Regulating Cell Functions Owned by Ligand Construct)

Using the surface 5 of substrates for cell culture prepared in Example 7, and the surface 5 without immobilization of the ligand construct, the property of regulating cell functions was evaluated. The evaluation was carried out using as an index an ability of differentiating immature osteoblasts derived from mouse (MC3T3-E1 cell) into mature osteoblasts (ALP activity) by the ligand construct. The results of the ALP activity per 1 cell measured on the seventh day of the culture of MC3T3-E1 cells are shown in Table 11.

[Table 11]

| Kinds of Surface on Substrate for Cell Culture | ALP Activity (fmol/min/cell) |
|---|---|
| Surface 5 | 38 (28) |

*1 The numerical figure in parenthesis is a measurement result of ALP activity at a surface without immobilization of KDGEA-SP20-(C)GRGDS.

As shown in Table 11, MC3T3-E1 cells cultured at surface having KDGEA-SP20-(C)GRGDS showed a markedly amplified ALP activity, as compared to the cells cultured at a surface without having KDGEA-SP20-(C)GRGDS.

INDUSTRIAL APPLICABILITY

According to the present invention, a ligand construct capable of regulating the cell functions efficiently and with high reproducibility, and a substrate for cell culture immobilized with the construct are provided. The present invention significantly contributes in the fields of development assistance of a medicament by the efficiency of the test regarding pharmaceutical efficacy, pharmacology and toxicity of the agent, an animal experimentation substitute method, biosensors using cells, supplying functional cells for studies or transplantation, studies for searching the mechanisms for exhibition of the cell functions, cell (regenerative) therapy, diagnosis using cells from a patient, and manufacture of useful substances such as medicaments.

BRIEF DESCRIPTION OF THE DRAWINGS

[Figure 1] A schematic view showing ONE example of a synthesis process relating to a method for preparing KDGEA-SP20-(Bio)GRGDS having 20 nm (60mer) dsDNA as a spacer.

[Figure 2] A schematic view showing ONE example of a synthesis process relating to a method for preparing KDGEA-SP20-GRGDS having 20 nm (60mer) dsDNA as a spacer.

SEQUENCE FREE TEXT

SEQ ID NO: 1 shows a sequence for a ligand.
SEQ ID NO: 2 shows a sequence for a ligand.
SEQ ID NO: 3 shows a sequence for a ligand.
SEQ ID NO: 4 shows a sequence for a ligand.
SEQ ID NO: 5 shows a sequence for a ligand.
SEQ ID NO: 6 shows a sequence for a ligand.
SEQ ID NO: 7 shows a sequence for a ligand.
SEQ ID NO: 8 shows a sequence for a ligand.
SEQ ID NO: 9 shows a sequence for a ligand.
SEQ ID NO: 10 shows a sequence for a ligand.
SEQ ID NO: 11 shows a sequence for a ligand.
SEQ ID NO: 12 shows a sequence for a ligand.
SEQ ID NO: 13 shows a sequence for a ligand.
SEQ ID NO: 14 shows a sequence for a ligand.
SEQ ID NO: 15 shows a sequence for a ligand.

## Claims

1. A ligand construct comprising at least two, identical or different, ligand molecules which are capable of binding to a cell surface receptor, the ligand molecules being coupled via a spacer, wherein the ligand construct has at least one functional group for binding substrates.

2. The ligand construct according to claim 1, wherein the spacer has a length of from 1 to 300 nm.

3. The ligand construct according to claim 1 or 2, wherein the spacer comprises a nucleic acid.

4. The ligand construct according to claim 3, wherein the spacer comprises a plural nucleic acids, at least two, identical or different, ligand molecules are each bound to identical or different nucleic acid chains.

5. The ligand construct according to claim 3 or 4, wherein the nucleic acid is deoxyribonucleic acid, ribonucleic acid, peptide nucleic acid, or a derivative thereof.

**6.** The ligand construct according to claim 5, wherein the ligand molecule is bound to 5'-terminal and/or 3'-terminal of deoxyribonucleic acid or a derivative thereof.

**7.** The ligand construct according to any one of claims 1 to 6, wherein the functional group for binding substrates is biotin or a derivative thereof.

**8.** A cell culture substrate comprising the ligand construct as defined in any one of claims 1 to 7 and a substrate moiety, wherein the ligand construct is immobilized to the substrate moiety via a functional group for binding substrates.

**9.** The cell culture substrate according to claim 8, wherein the substrate moiety comprises avidin, streptavidin or a derivative thereof.

**10.** The cell culture substrate according to claim 8 or 9, wherein the ligand construct is immobilized in an amount of from 0.1 fmol to 166 x $10^3$ fmol per 1 $cm^2$ of the substrate surface.

[Figure 1]

[Figure 2]

## INTERNATIONAL SEARCH REPORT

| | International application No. |
|---|---|
| | PCT/JP2006/325704 |

A. CLASSIFICATION OF SUBJECT MATTER
*C12N5/00*(2006.01)i, *C12N5/06*(2006.01)i, *C07K7/04*(2006.01)n

According to International Patent Classification (IPC) or to both national classification and IPC

B. FIELDS SEARCHED

Minimum documentation searched (classification system followed by classification symbols)
C12N5/00, C12N5/06, C07K7/04

Documentation searched other than minimum documentation to the extent that such documents are included in the fields searched

| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
BIOSIS/MEDLINE/WPIDS(STN), JMEDPlus(JDream2), JSTPlus(JDream2)

C. DOCUMENTS CONSIDERED TO BE RELEVANT

| Category* | Citation of document, with indication, where appropriate, of the relevant passages | Relevant to claim No. |
|---|---|---|
| X/A | JP 2005-535329 A  (Yale University),<br>24 November, 2005 (24.11.05),<br>Particularly, page 17, Par. Nos. [0077] to [0079]<br>& WO 2004/014311 A2      & WO 2005/016955 A2<br>& EP 1534736 A2          & EP 1660517 A2<br>& US 2005/0271655 A1 | 1-7/<br>8-10 |
| P,Y | JP 2006-42811 A  (Kuraray Co., Ltd.),<br>16 February, 2006 (16.02.06),<br>& JP 2006-42812 A        & JP 2006-45206 A<br>& JP 2006-45207 A | 1-10 |

☒ Further documents are listed in the continuation of Box C.   ☐ See patent family annex.

| | |
|---|---|
| * Special categories of cited documents:<br>"A" document defining the general state of the art which is not considered  to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 07 March, 2007 (07.03.07) | 13 March, 2007 (13.03.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.

PCT/JP2006/325704

C (Continuation). DOCUMENTS CONSIDERED TO BE RELEVANT

| Category* | Citation of document, with indication, where appropriate, of the relevant passages | Relevant to claim No. |
|---|---|---|
| P,Y | Toru NISHIMASA et al., "Avidin-Biotin Kagaku o Riyoshita Ondo Otosei Baiyozara Hyomen eno RGDS Peptide Kotei Hoho no Kento", The Society of Polymer Science, Japan (CD-ROM), 10 May, 2006 (10.05.06), Vol.55, No.1, 3J12 | 1-10 |
| A | WO 2003/002750 A2 (HIGH THROUGHPUT GENOMICS INC.), 09 January, 2005 (09.01.05), & JP 2005-525781 A    & JP 2001-526904 A & JP 2003-504011 A    & EP 1038033 A2 & EP 1190095 A2    & 1409739 A2 & US 6232066 B1    & US 6238869 B1 & US 6458333 B1    & US 9759198 B1 & WO 2000/037683 A2    & WO 2000/037684 A2 & 2000/079008 A2    & WO 99/032663 A2 | 1-10 |

Form PCT/ISA/210 (continuation of second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002355025 A
- JP 2002355026 A
- JP HEI7308186 B
- JP HEI11151086 B
- JP 2002355031 A
- JP 2002509001 A
- JP 2002505907 A

### Non-patent literature cited in the description

- Soyaku Saiensu no Susume-Posutogenomujidai no Paradaimu Shifuto. KYORITSU SHUPPAN CO., LTD, 2002
- **PP. LANZA ; R. LANGER ; J. VACANTI.** Principles of Tissue Engineering. Academic Press, 2000
- Saibogai Matorikkusu-Kiso to Rinsho. AICHI Shuppan Co., Ltd, 2000
- **Y. ITO.** *Biomaterials,* 1999, vol. 20, 2333
- **U. HERSEL ; C. DAHMEN ; H. KESSLER.** *Biomaterials,* 2003, vol. 24, 4385
- **H. SHIN ; S. JO ; AG. MIKOS.** *Biomaterials,* 2003, vol. 24, 4353
- **AS. BLAWAS ; WM. REICHERT.** *Biomaterials,* 1998, vol. 19, 595-609
- **RS. KANE ; S. TAKAYAMA ; E. OSTUNI ; DE. INGBER ; GM. WHITESIDES.** *Biomaterials,* 1999, vol. 20, 2363-2376
- **GA. DI LULLO ; SM. SWEENEY ; J. KORKKO ; L. ALAKOKKO ; JD. SAN ANTONIO.** *J. Biol. Chem.,* 2002, vol. 277 (6), 4223-4231
- **MJ. DALBY ; D. GIANNARAS ; MO. RIEHLE ; N. GADEGAARD ; S. AFFROSSMAN ; ASG. CURTIS.** *Biomaterials,* 2004, vol. 25, 77-83
- **XM. MO ; CY. XU ; M. KOTAKI ; S. RAMAKRISHNA.** *Biomaterials,* 2004, vol. 25, 1883-1890
- **K-B. LEE ; S-J. PARK ; CA. MIRKIN ; JC. SMITH ; M. MRKSICH.** *Science,* 2002, vol. 295, 1702-1705
- **DL. WILSON ; R. MARTIN ; S. HONG ; M. CRONIN-GOLOMB ; CA. MIRKIN ; DL. KAPLAN.** *Proc. Nat. Acad. Sci., USA,* 2001, vol. 98 (24), 13660-13664
- **W. DAI ; J. BELT ; WM. SALTZMAN.** *Bio/Technology,* 1994, vol. 12, 797-801
- **S. YAMAMOTO ; Y. KANEDA ; N. OKADA ; S. NAKAGAWA ; K. KUBO ; S. INOUE ; M. MAEDA ; Y. YAMASHIRO ; K. KAWASAKI ; T. MAYUMI.** *Anti-Cancer Drugs,* 1994, vol. 5, 424-428
- **Y. SUZUKI ; K. HOJO ; I. OKAZAKI ; H. KAMATA ; M. SASAKI ; M. MAEDA ; M. NOMIZU ; Y. YAMAMOTO ; S. NAKAGAWA ; T. MAYUMI.** *Chem. Pharm. Bull,* 2002, vol. 50 (9), 1229-1232
- **M. MAEDA ; Y. IZUNO ; K. KAWASAKI ; Y. KANEDA ; Y. MU ; Y. TSUTSUMI ; KW. LEM ; T. MAYUMI.** *Biochem. Biophys. Res. Commun.,* 1997, vol. 241, 595-598
- **M. MAEDA ; K. KAWASAKI ; Y. MU ; H. KAMADA ; Y. TSUTSUMI ; TJ. SMITH ; T. MAYUMI.** *Biochem. Biophys. Res. Commun.,* 1998, vol. 248, 485-489
- Saibogai Matorikusu-Kiso to Rinsho-. AICHI Shuppan Co., Ltd, 2000
- **MASAO HAYASHI.** Jikken Igaku Baio Saiensu, Shin Saibosecchakubunshi no Sekai. YODOSHA CO., LTD, 2001
- Tasaibotai no Kouchiku to Saibo Secchaku Sisutemu. **KIYOTOSHI SEKIGUCHI ; SHINTARO SUZUKI.** Shireezu: Baiosaiensu Shinseiki. KYORITSU SHUPPAN CO., LTD, 2001, 8
- Saibo Secchaku Kenkyu no Saizensen-Sigunaru Dentatsu kara Gan Ten-I heno Kanyo made. Jikken Igaku Zokan. YODOSHA CO., LTD, 1996
- Saibogai Matorikusu-Kiso to Rinsho. AICHI Shuppan Co., Ltd, 2000
- **WD. STAATZ ; JJ. WALSH ; T. PEXTON ; SA. SANTORO.** *J. Biol. Chem.,* 1990, vol. 265 (9), 4778-4781
- **JA. EBLE ; R. GOLBIK ; K. MANN ; K. KUHN.** *EMBO J.,* 1993, vol. 12 (12), 4795-4802
- **EC. TSILIBARY ; AS. CHARONIS.** *J. Cell. Biol.,* 1986, vol. 103 (6), 2467-2473
- **SK. AKIYAMA ; E. HASEGAWA ; T. HASEGAWA ; KM. YAMADA.** *J. Biol. Chem.,* 1985, vol. 260 (24), 13256-13260
- **MOTOYOSHI NOMIZU.** *Tanpakushitsu Kakusan Kouso,* 2000, vol. 45 (15), 2475-2482
- **CD. REYES ; AJ. GARCIA.** *J. Biomed. Mater. Res.,* 2003, vol. 65A, 511-523

- **GA. SULYOK ; C. GIBSON ; SL. GOODMAN ; G. HOLZEMANN ; M. WIESNER ; H. KESSLER.** *J. Med. Chem.,* 2001, vol. 44 (12), 1938-1950
- **U. HERSEL ; C. DAHMEN ; H. KESSLER.** *Biomaterials,* 2003, vol. 24, 4385-4415
- **JA. HUBBELL.** *Bio/Technology,* 1995, vol. 13, 565-576
- **H. SHIN ; S. JO ; AG. MIKOS.** *Biomaterials,* 2003, vol. 24, 4353-4364
- **E. KOIVUNEN ; W. ARAP ; D. RAJOTTE ; J. LAHDENRANTA ; R. PASQUALINI.** *J. Nuclear Med.,* 1999, vol. 40 (5), 883-888
- **Y. SUZUKI ; M. TANIHARA ; K. SUZUKI ; A. SAITOU ; W. SUFAN ; Y. NISHIMURA.** *J. Biomed. Mater. Res.,* 2000, vol. 50, 405-409
- **NC. WRINGTON ; FX. FARRELL ; R. CHANG ; AK. KASHYAP ; FP. BARBONE ; LS. MULCAHY ; DL. JOHNSON ; RW. BARRETT ; LK. JOLLIFFE ; WJ. DOWER.** *Science,* 1996, vol. 273, 458-463
- **A. VARKI.** *Proc. Natl. Acad. Sci. USA.,* 1994, vol. 91, 7390-7397
- **PE. NIELSEN ; M. EGHOLM ; RH. BERG ; O. BUCHARDT.** *Science,* 1991, vol. 254, 1497-1500
- non-RI Jikken no Saishin Purotokohru-Keikou no Genri to Jissai: Idenshikaiseki kara Baioime-jingu made. Bessatsu Jikken Igaku. YODOSHA CO., LTD, 1999
- **TOYOZO TAKAHASHI.** DNA Puroubu-Gijutsu to Ohyou. CMC, 1988
- **TOYOZO TAKAHASHI.** DNA Puroubu II-Shingijutsu to Shintenkai-. CMC, 1990
- **K. MATSUURA ; T. YAMASHITA ; Y. IGAMI ; N. KIMIZUKA.** *Chem. Commun.,* 2003, 376
- **NC. SEEMAN.** *Trends in Biotechnology,* 1999, vol. 17, 437-443
- **CM. NIEMEYER.** *Current Opinion in Chemical Biology,* 2000, vol. 4, 609-618
- **JH. GU ; LT. CAI ; S. TANAKA ; Y. OTSUKA ; H. TABATA ; T. KAWAI.** *J. Appl. Phys.,* 2002, vol. 92, 2816
- Tanpakushitu no Kagakushushoku <Jo>. **MOTONORI OHNO ; YUICHI KANEOKA ; FUMIO SAKIYAMA ; HIROSHI MAEDA.** Seibutsukagaku Jikken Ho. Japan Scientific Societies Press, 1981, 12
- Peptide Synthesis Protocols. Methods in Molecular Biology. Humana Press, 1994, 35
- Tousa Kougaku. Baiotekunoroji Johou Sentah. 1992
- Koubunshi Kagaku to Kakusan no Kino Dezain. Baio Koubunshi Kenkyuho. Japan Scientific Societies Press, 1996, 6
- **Y. ITO ; E. FUKUSAKI.** *J. Mol. Catal. B: Enzymatic,* 2004, vol. 28, 155-166
- Tanpakushitu no Kagakushushoku <Jo>. **NOBUYUKI YAMAZAKI ; MOTONORI OHNO ; YUICHI KANEOKA ; FUMIO SAKIYAMA ; HIROSHI MAEDA.** Seibutsukagaku Jikken Ho. Japan Scientific Societies Press, 1981, 12
- **RS. KANE et al.** *Biomaterials,* 1999, vol. 20, 2363-2376
- Geru Handobukku. NTN, 1997
- **K. SATO ; A. HIBARA ; M. TOKESHI ; H. HISAMOTO ; T. KITAMORI.** *Analytical Sciences,* 2003, vol. 19, 15-22
- **SP. MASSIA ; JA. HUBBELL.** *J. Cell Biol.,* 1991, vol. 114 (5), 1089-1100
- Soshiki Baiyo no Gijutsu. Asakura Shoten, 581